# EUROPEAN PATENT SPECIFICATION

(11) **EP 1 761 514 B1**
(45) Date of publication and mention of the grant of the patent: **30.07.2008**
(21) Application number: 05752829.1
(22) Date of filing: 13.06.2005
(51) Int. Cl.: C07D 307/93, A61K 31/34, A61P 25/00

(54) **NOVEL TETRACYCLIC TETRAHYDROFURAN DERIVATIVES**
NEUE TETRACYCLISCHE TETRAHYDROFURANDERIVATE
NOUVEAUX DERIVES TETRACYCLIQUES DE TETRAHYDROFURANE

(30) Priority: 14.06.2004 WO PCT/EP2004/051105
(43) Date of publication of application: 14.03.2007
(73) Proprietor: Janssen Pharmaceutica NV, 2340 Beerse (BE)
(72) Inventor: CID-NUNEZ, José Maria Johnson & Johnson Pharmaceutical Research and Development Division of Janssen-Cilag,S.A., 45007 Toledo (ES); MEGENS, Antonius Adrianus Hendrikus Petrus, 2340 Beerse (BE); TRABANCO-SUÁREZ, Andrés Avelino Johnson & Johnson Pharmaceutical Research and Development Division of Janssen-Cilag,S.A., 45007 Toledo (ES)
(74) Representative: Daelemans, Frank F.R.
(86) International application number: PCT/EP2005/052706
(87) International publication number: WO 2005/121113

(56) References cited:
- WO-A-97/38991
- WO-A-99/19317
- WO-A-03/048146

## Description

### Field of the Invention

This invention concerns novel substituted tetracyclic tetrahydrofuran derivatives with binding affinities towards serotonin receptors, in particular 5-HT_{2A} and 5-HT_{2C} receptors, and towards dopamine receptors, in particular dopamine D₂ receptors and with norepinephrine (NE) reuptake inhibition properties, pharmaceutical compositions comprising the compounds according to the invention, the use thereof as a medicine, in particular for the prevention and/or treatment of a range of psychiatric and neurological disorders, in particular certain psychotic, cardiovascular and gastrokinetic disorders and processes for their production.

### Background Prior Art

WO 97/38991, published October 23, 1997 (Janssen Pharmaceutica N.V.) discloses substituted tetracyclic tetrahydrofuran derivatives that may be used as therapeutic agents in the treatment or prevention of CNS disorders, cardiovascular disorders or gastrointestinal disorders. In particular, the compounds show affinity for the serotonin 5-HT₂ receptors, particularly for the 5-HT_{2A} and 5-HT_{2C}-receptors.

WO 99/19317, published April 22, 1999 (Janssen Pharmaceutica N.V.) discloses substituted tetracyclic tetrahydrofuran derivatives with a specific halogen substitution pattern on the dibenzoazepine, dibenzooxepine, dibenzothiepine or dibenzosuberane ring. The compounds are useful in the treatment or prevention of CNS disorders, cardiovascular disorders or gastrointestinal disorders and show a faster onset of action over the compounds as disclosed in WO 97/38991.

Both WO 03/048146, published June 12, 2003 (Janssen Pharmaceutica N.V.) and WO 03/048147, published June 12, 2003 (Janssen Pharmaceutica N.V.) disclose processes for the preparation of each of the four diastereomers of trans-, respectively cis-fused 3,3a,8,12b-tetrahydro-2*H*-dibenzo[3,4:6,7]cyclohepta[1,2-b]furan derivatives in a stereochemically pure form from a single enantiomerically pure precursor. The compounds of WO 03/048146 show affinity for 5-HT₂ receptors, particularly for 5-HT_{2A} and 5-HT_{2C} receptors. The compounds of WO 03/048147 show affinity for the serotonin 5-HT_{2A}, 5-HT_{2C} and 5-HT₇ receptors , the H₁-receptors (pIC₅₀=7.15-7.89), D2 and/or D3 receptors and for the norepinephrine reuptake transporters (pIC₅₀ = 6.03-7.34). The compounds disclosed in the latter two publications do not contain a cyclic amine side chain.

WO 03/040122, published May 15, 2003 (Janssen Pharmaceutica N.V.) discloses mandelate salts of the compounds according to WO 97/38991 and WO 99/19317. Said salts were surprisingly found to be more stable at enhanced temperature and relative humidity than the compounds disclosed in WO 97/38991 and WO 99/19317.

### Background of the Invention

Compounds of the type specifically described in the above patent filings generally have broad spectrum psychotropic properties with atypical antipsychotic, anxiolytic, antidepressant and socialising properties with a complex pharmacological profile. Thus, such compounds typically have an affinity for the 5-HT_{2A} and 5-HT_{2C} receptors, central 5-HT_{2A} antagonism being known to improve the negative symptoms of schizophrenia, while central 5-HT_{2C} antagonism is believed to contribute to anxiolytic and disinhibitory properties. The compounds also generally have affinity for the D₂ receptor, central D₂ antagonism being effective against mania, excitement, aggression, and the positive symptoms of schizophrenia. Finally the compounds have inhibitory effects against norepinephrine uptake, i.e. an affinity for the norepinephrine transporter (NET) which contributes to antidepressant activity. It is notable that the compounds specifically described in the above filings are structurally characterised by a methylene group or an oxygen heteroatom in the 8-position bridging the benzene rings.

We have now discovered a narrow subclass of tetracyclic tetrahydrofuran derivatives which are characterised by the presence of certain substituting groups on the bridging 8-carbon atom, in contrast to the compounds specifically described in the above filings which have either a heteroatom or a bridging methylene group in this position. The presence of such substituting groups in the compounds provides an improved balance of properties for the treatment of depression, namely a higher inhibitory effect against norepinephrine uptake and a low antagonist effect against the D₂ receptor (lower D₂/NET ratio). It should be noted that the latter antagonist effect, while generally lower than those the compounds in the earlier filings, is still significant and contributes to the useful pharmacological profile of the compounds of the present invention. In addition the compounds of the invention have been found to have improved metabolic stability, as measured by the human liver microsome assay. which may be useful for example in providing compounds with a longer effective half life and hence longer duration of action.

### Description of the Invention

It was the object of the present invention to provide compounds falling within the scope of the above WO 97/38991 filing, but not disclosed therein, which have surprising advantageous properties over the compounds particularly described in the said filing

This goal was achieved by the present novel compounds according to Formula (I) : 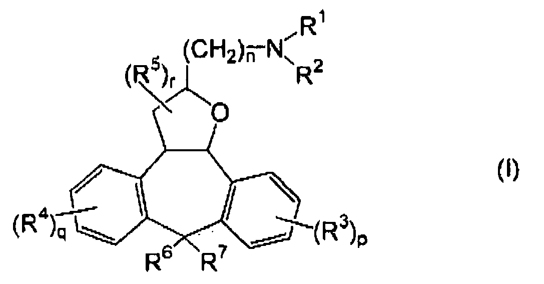 a pharmaceutically acceptable acid or base addition salt thereof, a stereochemically isomeric form thereof and an *N*-oxide form thereof, wherein :
- n: is an integer equal to zero, 1, 2, 3, 4, 5, or 6;
- p: is an integer equal to zero, 1, 2, 3 or 4;
- q: is an integer equal to zero, 1, 2, 3 or 4;
- r: is an integer equal to zero, 1, 2, 3, 4 or 5;
- R¹ and R²: each independently is selected from the group of hydrogen; C₁₋₆alkyl; C₁₋₆alkylcarbonyl; halomethylcarbonyl; aryl ; alkylsulphonyl and C₁₋₆alkyl substituted with hydroxy, C₁₋₆alkyloxy, carboxyl, C₁₋₆alkylcarbonyloxy, C₁₋₆alkyloxycarbonyl or aryl;
or R¹ and R² taken together with the nitrogen atom to which they are attached may form a morpholinyl ring or a radical of Formula (a) to (e): 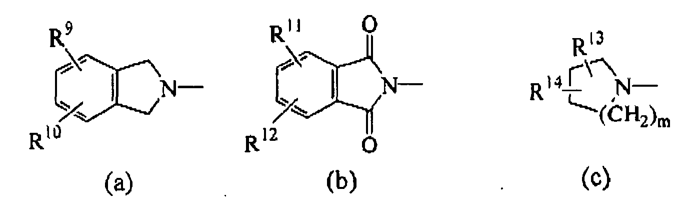 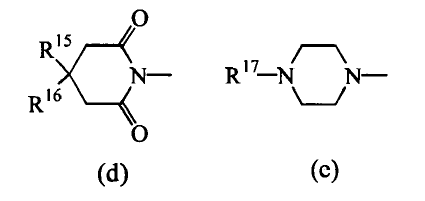 wherein:
- R9, R¹⁰, R¹¹ and R¹²: each independently are selected from the group of hydrogen ; halo ; halomethyl and C₁₋₆alkyl;
- m: is an integer equal to zero, 1, 2, or 3;
- R¹³, R¹⁴, R¹⁵ and R¹⁶: each independently are selected from the group of hydrogen ; C₁₋₆alkyl ; aryl and arylcarbonyl; or R¹⁵ and R¹⁶ taken together may form a bivalent radical C₄₋₅alkanediyl ;
- R¹⁷: is selected from the group of hydrogen; C₁₋₆alkyl; C₁₋₆alkylcarbonyl; halomethylcarbonyl; C₁₋₆alkyloxycarbonyl; aryl; di(aryl)methyl; C₁₋₆alkyl substituted with hydroxy, C₁₋₆alkyloxy, carboxyl, C₁₋₆alkylcarbonyloxy, C₁₋₆alkyloxycarbonyl and aryl;

- each R³: independently is selected from the group of hydrogen ; halo ; cyano ; hydroxy ;halomethyl ; halomethoxy ; carboxyl; nitro; amino ; mono- or di(C₁₋₆alkyl)amino ; C₁₋₆alkylcarbonylamino aminosulfonyl ; mono- or di(C₁₋₆alkyl)aminosulfonyl ; C₁₋₆alkyl ; C₁₋₆alkyloxy ; C₁₋₆alkylcarbonyl and C₁₋₆alkyloxycarbonyl;
- each R⁴: independently is selected from the group of hydrogen ; halo ; cyano ; hydroxy ; halomethyl ; halomethoxy ; carboxyl ; nitro ; amino ; mono- or di(C₁₋₆alkyl)amino ; C₁₋₆alkylcarbonylamino ; aminosulfonyl ; mono- or di(C₁₋₆alkyl)aminosulfonyl ; C₁₋₆alkyl ; C₁₋₆alkyloxy ; C₁₋₆alkylcarbonyl and C₁₋₆alkyloxycarbonyl;
- each R⁵: independently is selected from the group of C₁₋₆alkyl ; cyano and halomethyl;
- R⁶ and R⁷: each independently from each other, are selected from the group of hydrogen, hydroxy, C₁₋₆alkyl, halomethyl and C₁₋₆alkyloxy ; with the proviso that R⁶ and R⁷ are not simultaneously hydrogen ; or R⁶ and R⁷ taken together may form a methylene radical (=CH₂); or, together with the carbon atom to which they are attached, a carbonyl; and
- aryl: is phenyl; or phenyl substituted with 1, 2 or 3 substituents selected from the group of halo, hydroxy, C₁₋₆alkyl and halomethyl.

More in particular, the invention relates to a compound according to the general Formula (1), a pharmaceutically acceptable acid or base addition salt thereof, a stereochemically isomeric form thereof and an *N*-oxide form thereof, wherein n is 1.

More in particular, the invention relates to a compound according to the general Formula (I), a pharmaceutically acceptable acid or base addition salt thereof, a stereochemically isomeric form thereof and an *N*-oxide form thereof, wherein :
- n: is an integer equal to 1;
- p: is an integer equal to zero or 1;
- q: is an integer equal to zero or 1;
- r: is an integer equal to zero;
- R¹ and R² e: ach independently is selected from the group of hydrogen ; C₁₋₆alkyl ; aryl; alkylsulphonyl and C₁₋₆alkyl substituted with carboxyl or aryl ;
- R³: is selected from the group of hydrogen ; halo ; amino ; mono- or di(C₁₋₆alkyl)amino and C₁₋₆alkyloxy;
- R⁴: is hydrogen or halo;
- R⁶ and R⁷: each independently from each other, are selected from the group of hydrogen ; hydroxy ; C₁₋₆alkyl and C₁₋₆alkyloxy ; with the proviso that R⁶ and R⁷ are not simultaneously hydrogen ; or R⁶ and R⁷ taken together form methylene (=CH₂); or, together with the carbon atom to which they are attached, a carbonyl.

More in particular, the invention relates to a compound according to the general Formula (I), a pharmaceutically acceptable acid or base addition salt thereof, a stereochemically isomeric form thereof and an *N-oxide* form thereof, wherein R¹ and R² are each independently selected from the group of hydrogen ; methyl; ethyl ; methoxy ; phenyl and benzyl.

More in particular, the invention relates to a compound according to the general Formula (I), a pharmaceutically acceptable acid or base addition salt thereof, a stereochemically isomeric form thereof, and an *N*-oxide form thereof, wherein both R¹ and R² are methyl ; or R is hydrogen and R² is methyl.

More in particular, the invention relates to a compound according to the general Formula (I), a pharmaceutically acceptable acid or base addition salt thereof, a stereochemically isomeric form thereof and an *N*-oxide form thereof, wherein p is zero or 1 and R³ is selected from the group of hydrogen ; fluoro ; chloro ; bromo ; methoxy ; amino ; methylamino and dimethylamino.

More in particular, the invention relates to a compound according to the general Formula (I), a pharmaceutically acceptable acid or base addition salt thereof, a stereochemically isomeric form thereof and an *N*-oxide form thereof, wherein q is zero or 1 and R⁴ is selected from the group of hydrogen and fluoro.

Preferred compounds are those particular compounds according to the invention wherein R⁶ and R⁷ are selected from the group of hydrogen, methyl, ethyl, isopropyl, hydroxy, methoxy and isopropoxy; or R⁶ and R⁷ taken together may form methylene; or, together with the carbon atom to which they are attached, a carbonyl.

Preferred compounds are also those particular compounds according to the invention wherein the hydrogen atoms on carbon atoms 3a and 12b have a trans configuration or those compounds having the(2α, 3aα, 12bβ) stereochemical configuration.

Preferred compounds are also those compounds according to the invention where the compounds are selected from the group of compounds :
ο (5,11-difluoro-8-methylene-3,3a,8,12b-teuahydro-2*H*-1-oxa-dibenzo[e,h]azulen-2-ylmethyl)-dimethyl-amine ;
ο 11-fluoro-2-methylaminomethyl-3,3a,8,12b-tetrahydro-2*H*-1-oxa-dibenzo[e,h]-azulen-8-ol ;
ο 11-fluoro-8-methyl-2-methylaminomethyl-3,3a,8,12b-tetrahydro-2*H*-1-oxa-dibenzo-[e,h]azulen-8-ol;
ο 2-dimethylaminomethyl-8-methyl-3,3a,8,12b-tetrahydro-2*H*-1-oxa-dibenzo[e,h]-azulen-8-ol;
ο (11-fluoro-8-methoxy-8-methyl-3,3a,8,12b-tetrahydro-2*H*-1-oxa-dibenzo[e,h]azulen-2-ylmethyl)-dimethyl-amine;
ο (11-fluoro-8-methyl-3,3a,8,12b-tetrahydro-2*H*-1-oxa-dibenzo[e,h]azulen-2-ylmethyl)-dimethyl-amine;
ο (8-methyl-3,3a,8,12b-tetrahydro-2*H*-1-oxa-dibenzo[e,h]azulen-2-ylmethyl)-dimethyl-amine; and
ο (8-methyl-3,3a,8,12b-tetrahydro-2*H*-1-oxa-dibenzo[e,h]azulen-2-ylmethyl)-methylamine.

Most preferred compounds are also those compounds according to the invention where the compounds are selected from the group of compounds defined by the compound numbers 27, 29, 34, 45, 66 and 74 as disclosed in the application, in particular in Tables 1 and 2.

In the framework of this application alkyl defines straight and branch chained saturated hydrocarbon radicals having from 1 to 6 carbon atoms such as, for example, methyl, ethyl, propyl, butyl, 1-methylpropyl, 1,1-dimethylethyl, pentyl, hexyl; C₄₋₅alkanediyl defines bivalent straight and branch chained saturated hydrocarbon radicals having from 4 to 5 carbon atoms such as, for example, 1,4-butanediyl, 1,5-pentanediyl; halo is generic to fluoro, chloro, bromo and iodo.

In the framework of this application, the term halomethyl is meant to include mono-, di-, and trihalomethyl. Examples of halomethyl are fluoromethyl, difluoromethyl and particularly trifluoromethyl.

The pharmaceutically acceptable salts are defined to comprise the therapeutically active non-toxic acid addition salts forms that the compounds according to Formula (I) are able to form. Said salts can be obtained by treating the base form of the compounds according to Formula (I) with appropriate acids, for example inorganic acids, for example hydrohalic acid, in particular hydrochloric acid, hydrobromic acid, sulfuric acid, nitric acid and phosphoric acid; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicylic acid, p-aminosalicylic acid, pamoic acid or mandelic acid.
The compounds according to Formula (I) containing acidic protons may also be converted into their therapeutically active non-toxic metal or amine addition salts forms by treatment with appropriate organic and inorganic bases. Appropriate base salts forms comprise, for example, the ammonium salts, the alkaline and earth alkaline metal salts, in particular lithium, sodium, potassium, magnesium and calcium salts, salts with organic bases, e.g. the benzathine, *N*-methyl-D-glucamine, hybramine salts, and salts with amino acids, for example arginine and lysine.
Conversely, said salts forms can be converted into the free forms by treatment with an appropriate base or acid.

The term addition salt as used in the framework of this application also comprises the solvates that the compounds according to Formula (I) as well as the salts thereof, are able to form. Such solvates are, for example, hydrates and alcoholates.

The *N*-oxide forms of the compounds according to Formula (I) are meant to comprise those compounds of Formula (I) wherein one or several nitrogen atoms are oxidized to the so-called *N*-oxide, particularly those *N*-oxides wherein one or more tertiary nitrogens (e.g. particularly those tertiary nitrogens bearing the R¹ and R² substituents) are *N*-oxidized. Such *N*-oxides can easily be obtained by a skilled person without any inventive skills and they are obvious alternatives for the compounds according to Formula (I) since these compounds are metabolites, which are formed by oxidation in the human body upon uptake. As is generally known, oxidation is normally the first step involved in drug metabolism (Textbook of Organic Medicinal and Pharmaceutical Chemistry, 1977, pages 70- 75). As is also generally known, the metabolite form of a compound can also be administered to a human instead of the compound per se, with much the same effects.

The compounds of Formula (I) may be converted to the corresponding *N*-oxide forms following art-known procedures for converting a trivalent nitrogen into its *N*-oxide form. Said *N*-oxidation reaction may generally be carried out by reacting the starting material of Formula (I) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. *tert-*butyl hydroperoxide. Suitable solvents are, for example, water, lower alkanols, e.g. ethanol and the like, hydrocarbons, e.g. toluene, ketones, e.g. 2-butanone, halogenated hydrocarbons, e.g. dichloromethane, and mixtures of such solvents.

The term "stereochemically isomeric forms" as used hereinbefore defines all the possible isomeric forms that the compounds of Formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Compounds encompassing double bonds can have an E or Z-stereochemistry at said double bond. Stereochemically isomeric forms of the compounds of Formula (I) are obviously intended to be embraced within the scope of this invention.

Following CAS nomenclature conventions, when two stereogenic centers of known absolute configuration are present in a molecule, an R or S descriptor is assigned (based on Cahn-Ingold-Prelog sequence rule) to the lowest-numbered chiral center, the reference center. R* and S* each indicate optically pure stereogenic centers with undetermined absolute configuration. If "α" and "β" are used: the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system (hydrogen atom in compounds according to Formula (I)) relative to the position of the highest priority substituent on the reference atom is denominated "α", if it is on the same side of the mean plane determined by the ring system, or "β", if it is on the other side of the mean plane determined by the ring system.

The numbering of the tetracyclic ring-system present in the compounds of Formula (I), as defined by Chemical Abstracts nomenclature is shown in the Formula below.

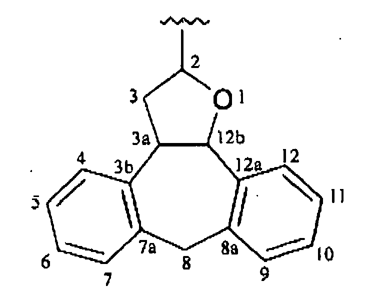

The compounds of Formula (I) have at least three stereogenic centers in their chemical structure, namely carbon atom 2, 3a and 12b. Said asymmetric center and any other asymmetric center which may be present, are indicated by the descriptors R and S.

The compounds of Formula (I) as prepared in the processes described below may be synthesized in the form of racemic mixtures of enantiomers that can be separated from one another following art-known resolution procedures. The racemic compounds of Formula (I) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (1) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

### Pharmacology

The compounds of the present invention show affinity for 5-HT₂ receptors, particularly for 5-HT_{2A} and 5-HT_{2C} receptors (nomenclature as described by D. Hoyer in "Serotonin (5-HT) in neurologic and psychiatric disorders" edited by M.D. Ferrari and published in 1994 by the Boerhaave Commission of the University of Leiden). The serotonin antagonistic properties of the present compounds may be demonstrated by their inhibitory effect in the "5-hydroxytryptophan Test on Rats" which is described in Drug Dev. Res., 13, 237-244 (1988). Furthermore, compounds of the present invention show interesting pharmacological activity in the "mCPP Test on Rats", and in the "Combined Apomorphine, Tryptamine, Norepinephrine (ATN) Test on Rats" which is described in Arch. Int. Pharmacodyn, 227, 238-253 (1977). The compounds also show affinity for the D₂ receptor and for the norepinephrine transporter, as demonstrated by the results of the assays described below.

Also, the compounds of the present invention have favourable physicochemical properties. For instance, they are chemically stable compounds.

In view of their capability to antagonize or interfere with the actions of serotonin, dopamine and norepinephrine, the compounds according to the invention are useful as a medicine, in particular in the prophylactic and/or therapeutic treatment of serotonin, dopamine and norepinephrine-mediated conditions.
The invention therefore relates to a compound according to the general Formula (I), a pharmaceutically acceptable acid or base addition salt thereof, a stereochemically isomeric form thereof and an *N*-oxide form thereof, for use as a medicine.

The invention therefore also relates to the use of a compound according to the general Formula (I), a pharmaceutically acceptable acid or base addition salt thereof, a stereochemically isomeric form thereof and an *N*-oxide form thereof for the manufacture of a medicament for the prophylactic and/or therapeutic treatment of serotonin-, dopamine- and norepinephrine-mediated conditions.

More in particular, the compounds of Formula (I), a pharmaceutically acceptable acid or base addition salt thereof, a stereochemically isomeric form thereof and an *N*-oxide form thereof, are useful as therapeutic agents in the prophylactic and/or therapeutic treatment of central nervous system disorders, in particular anxiety, depression and mild depression, bipolar disorders including bipolar mania and depression, sleep- and sexual disorders, psychosis, borderline psychosis, schizophrenia, migraine, personality disorders or obsessive-compulsive disorders, autism, social phobias or panic attacks, attention disorders including attention deficit hyperactivity disorder (ADHD), organic mental disorders, mental disorders in children, aggression, memory disorders and attitude disorders in older people, addiction, obesity, bulimia and similar disorders. In particular, the present compounds may be used as anxiolytics, antipsychotics, antidepressants, anti-migraine agents and as agents having the potential to overrule the addictive properties of drugs of abuse.

More in particular, the compounds of Formula (I), a pharmaceutically acceptable acid or base addition salt thereof, a stereochemically isomeric form thereof and an *N*-oxide form thereof may also be used as therapeutic agents in the treatment of motoric disorders. It may be advantageous to use the present compounds in combination with classical therapeutic agents for such disorders.

More in particular, the compounds of Formula (I), a pharmaceutically acceptable acid or base addition salt thereof, a stereochemically isomeric form thereof and an *N*-oxide form thereof, may also serve in the treatment or the prevention of damage to the nervous system caused by trauma, stroke, neurodegenerative illnesses, cognitive disorders such as dementia and Alzheimers disease, and the like; cardiovascular disorders like high blood pressure, thrombosis, stroke, and the like; and gastrointestinal disorders like dysfunction of the motility of the gastrointestinal system and the like.

Most in particular, the compounds of Formula (I), a pharmaceutically acceptable acid or base addition salt thereof, a stereochemically isomeric form thereof and an *N*-oxide form thereof, may be used for the treatment and/or prophylaxis of anxiety, psychosis, depression, bipolar disorders including bipolar depression, migraine and addictive properties of drugs of abuse.

In view of the above uses of the compounds of Formula (I), it follows that the present invention also provides a method of treating warm-blooded animals suffering from such diseases, said method comprising the systemic administration of a therapeutic amount of a compound of Formula (I), a pharmaceutically acceptable acid or base addition salt thereof, a stereochemically isomeric form thereof and an *N*-oxide form thereof effective in treating the above described disorders, in particular, in treating anxiety, psychosis, depression, migraine and addictive properties of drugs of abuse.

Those skilled in the treatment of such diseases could determine the effective therapeutic daily amount from the test results presented hereinafter. An effective therapeutic daily amount would be from about 0.01 mg/kg to about 10 mg/kg body weight, more preferably from about 0.05 mg/kg to about 1 mg/kg body weight.

The compounds according to the invention, in particular the compounds according to Formula (I), the pharmaceutically acceptable acid or base addition salts thereof, the stereochemically isomeric forms thereof and the N-oxide form thereof, or any subgroup or combination thereof may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally, rectally, percutaneously, by parenteral injection or by inhalation. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations that are intended to be convened, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. Said additives may facilitate the administration to the skin and/or may be helpful for preparing the desired compositions. These compositions may be administered in various ways, e.g., as a transdermal patch, as a spot-on, as an ointment.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

The invention also relates to a pharmaceutical composition comprising a pharmaceutically acceptable carrier and, as active ingredient, a therapeutically effective amount of a compound according to the invention, in particular a compound according to Formula (I), the pharmaceutically acceptable acid or base addition salts thereof, the stereochemically isomeric forms thereof and the *N*-oxide form thereof.

For ease of administration, the subject compounds may be formulated into various pharmaceutical forms for administration purposes. To prepare the pharmaceutical compositions of this invention, a therapeutically effective amount of the particular compound, optionally in addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable solutions containing compounds of Formula (I) may be formulated in an oil for prolonged action. Appropriate oils for this purpose are, for example, peanut oil, sesame oil, cottonseed oil, corn oil, soybean oil, synthetic glycerol esters of long chain fatty acids and mixtures of these and other oils. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment. Acid or base addition salts of compounds of Formula (I) due to their increased water solubility over the corresponding base or acid form, are more suitable in the preparation of aqueous compositions.
In order to enhance the solubility and/or the stability of the compounds of Formula (I) in pharmaceutical compositions, it can be advantageous to employ α-, β- or γ-cyclodextrins or their derivatives, in particular hydroxyalkyl substituted cyclodextrins, e.g. 2-hydroxypropyl-β-cyclodextrin. Also co-solvents such as alcohols may improve the solubility and/or the stability of the compounds of Formula (I) in pharmaceutical compositions.

### Synthesis

The compounds according to the invention can generally be prepared by a succession of steps, each of which is known to the skilled person. The route shown in the following reaction scheme 1 may be used for the preparation of compounds of Formula (I) in which R⁶ is hydrogen and R⁷ is C₁₋₆alkyl, said compounds being represented by Formula (I-a).

In reaction scheme 1, W is an hydroxy group, a protected hydroxy group or a leaving group such as halo, benzyloxy, benzoyloxy or a sulphonyloxy group such as p-toluenesulphonyloxy, methanesulphonyloxy or trifluoromethanesulphonyloxy; and R^{7a} is hydrogen or C₁₋₆alkyl. 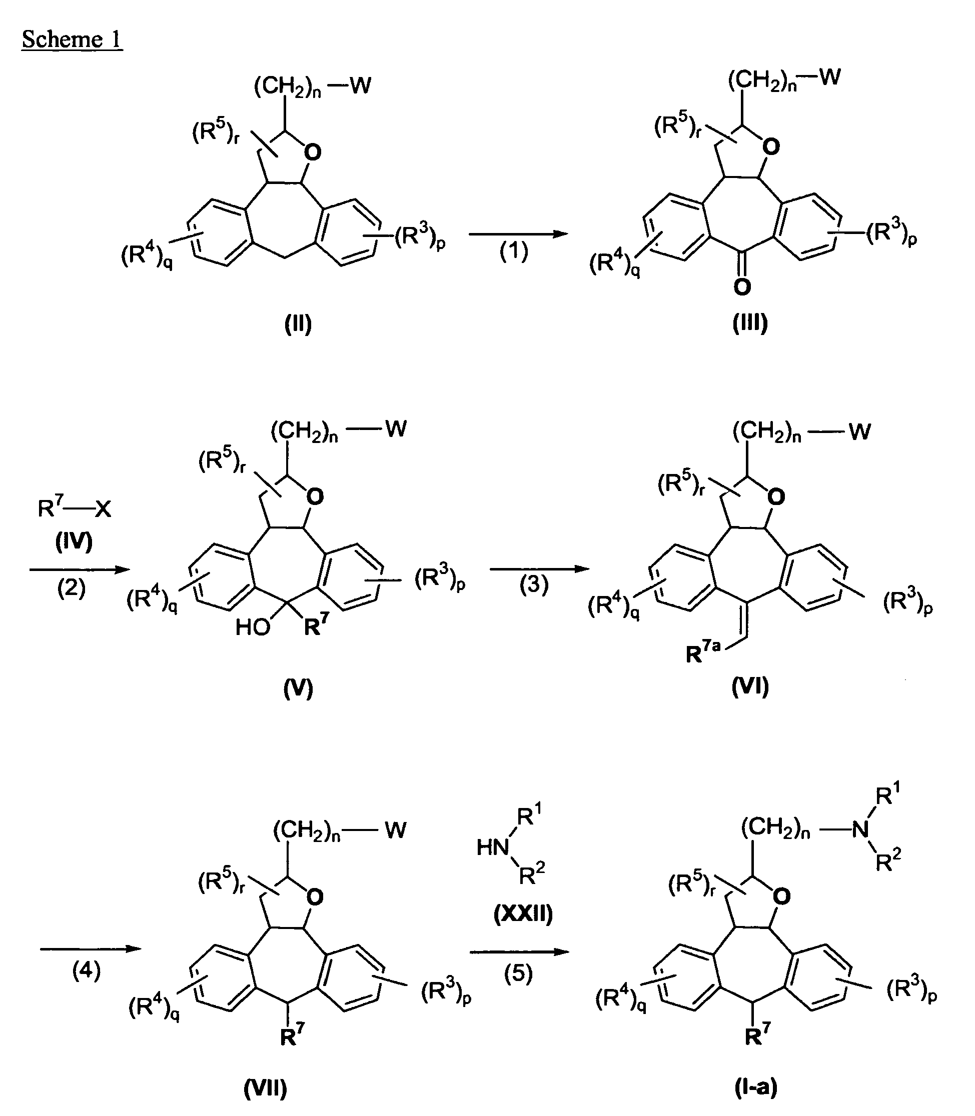
Step 1: Oxidation of a compound of Formula (II) to form a ketone of Formula (III). The oxidation is effected by a suitable oxidising reagent such as KMnO₄ or CrO₃. The oxidation reaction can be effected for example under basic conditions e.g. aqueous potassium hydroxide, in an organic solvent such as dichloromethane, and in the presence of HSO₃NBu₄ at room temperature.
Step 2: Reaction of a ketone compound of Formula (III) with an organometallic reagent of Formula R⁷-X (Formula IV) where X is for example MgCl, to form a C8-(hydroxy)-C8-(R⁷)-disubstituted compound of Formula (V). The reaction is generally effected in an organic solvent such as tetrahydrofuran.
Step 3: Dehydration of a compound of Formula (V) to form a methylene-substituted compound of Formula (VI). The dehydration may be effected, for example, by treatment with SOCl₂ in a basic medium such as pyridine, for example at a temperature from 0°C to room temperature.
Step 4: Hydrogenation of a compound of Formula (VI) to form a R⁷-substituted compound of Formula (VII). The hydrogenation is conveniently carried out using hydrogen and a palladium on carbon catalyst in an organic solvent such as for example, methanol, isopropyl alcohol, acetone or ethyl acetate and under hydrogen pressure, as for example atmospheric pressure, and room temperature.
Step 5: *N*-alkylation of a compound of Formula (VII) with an amine of Formula (XXII). The *N*-alkylation can conveniently be carried out in a reaction-inert solvent such as, for example, methanol, tetrahydrofuran, methylisobutylketone, *N,N-*dimethylformamide, acetonitrile or dimethylsulfoxide, and optionally in the presence of a suitable base such as calcium oxide. Stirring and an elevated temperature, for instance a reflux temperature, may enhance the rate of the reaction. Alternatively, said *N*-alkylation may also be performed using the procedure described by Monkovic et al. (J. Med. Chem. (1973), 16(4), p. 403-407), which involves the use of a pressurised reaction vessel. Compounds of Formula (I) in which R⁶ is C₁₋₆alkyloxy may be prepared by treatment of a compound of Formula (V) as above with a C₁₋₆alkylating agent, for example a C₁₋₆ alkyl halide, in the presence of sodium hydride, and subsequent reaction with an amine of Formula (IX) in analogous manner.

The following route (Scheme 2) describes an alternative process for the preparation of compounds of Formula (I-a), also including the preparation of compounds of Formula (1) in which R⁶ and R⁷ together with the carbon to which they are attached, form a carbonyl group, represented by Formula (I-b), compounds of Formula (I) in which R⁶ is hydroxy and R⁷ is a C₁₋₆ alkyl group, represented by Formula (I-c), and compounds of Formula (I) in which R⁶ and R⁷ form a methylene group, represented by Formula (I-d), whereR^{7a} is hydrogen. 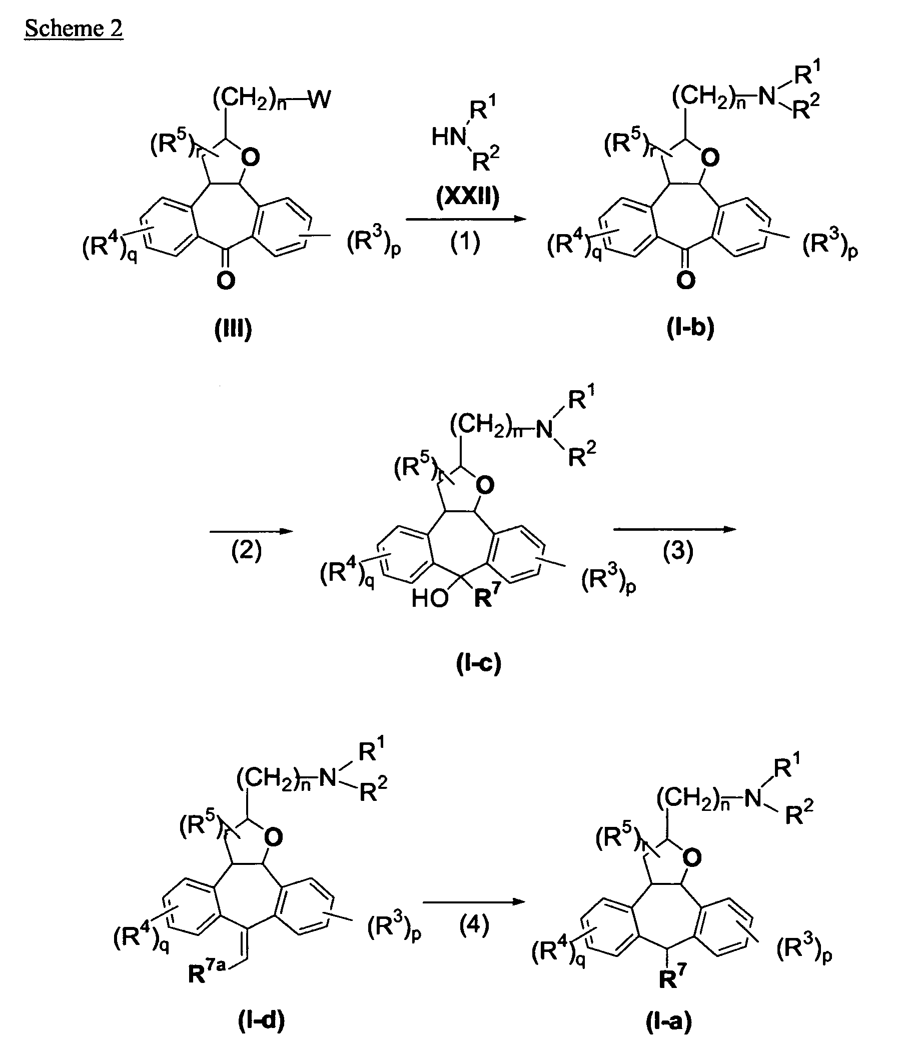 The steps in the above synthetic route can be effected in a similar manner to that described for the analogous steps in Scheme 1. 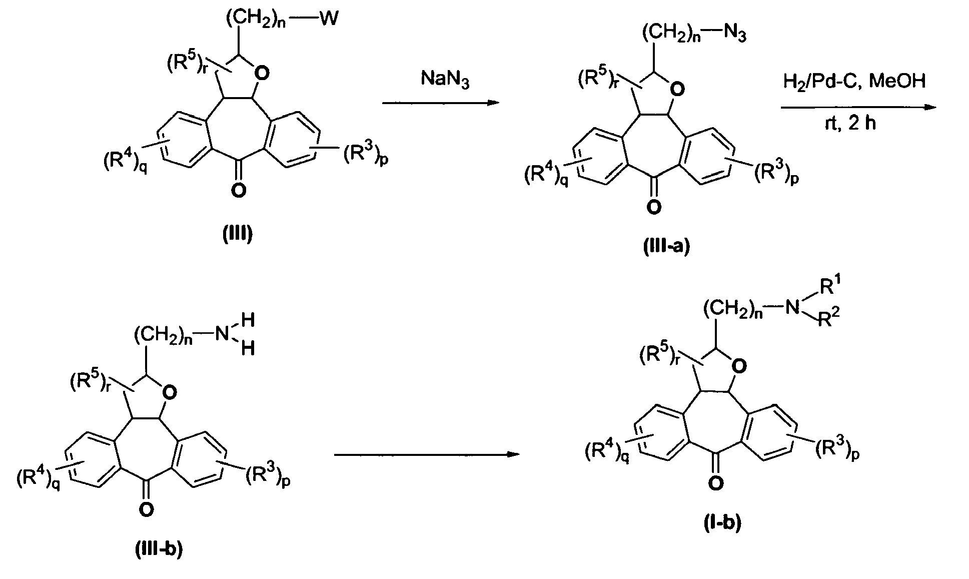 Alternatively, compounds of Formula (I-b) can be prepared by reaction of a compound of Formula (III), wherein W in Formula (III) is any leaving group, such as for example halo or 4-methylphenylsulphonyloxy. The preparation of the compound of Formula (III-a) is carried out by reacting a compound of Formula (III) with an azide, such as for example sodium azide (NaN₃), in presence of an organic solvent such as for example dimethylformamide, for example at 100 °C, to form the corresponding azido analog of Formula (III-a). A compound of Formula (III-b) can be prepared from a compound of Formula (III-a) for example by catalytic hydrogenation, e.g. using platinum or palladium on carbon under hydrogen atmosphere in a inert solvent such as methanol or alternatively using triphenylphosphine in an organic solvent such as methanol. A compound of Formula (I-b) can be prepared by a skilled person from a compound of Formula (III-b) following art known procedures such as alkylation of the nitrogen atom with a suitable alkylating reagent or by alkylation of the nitrogen atom by reductive amination reaction with a suitable carbonyl compound.

Alternatively, compounds of Formula (I-c) can be prepared by reaction of a compound of Formula (V) with an amine of Formula (XII) in a similar manner to that described for step 5 in Scheme 1.

Alternatively, compounds of Formula (I-d) can be prepared by reaction of a compound of Formula (VI) with an amine of Formula (XXII) in a similar manner to that described for step 5 in Scheme 1.

Alternatively, compounds of Formula (1-b) can be prepared according to Scheme 3 shown below:

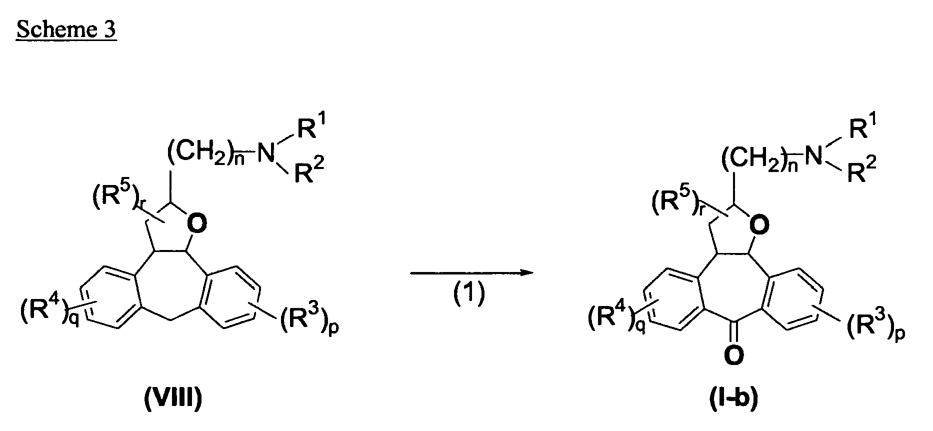

The above conversion can be effected in a similar manner to that described for the analogous step in Scheme 1 using preferably an oxidising reagent such as CrO₃ in refluxing acetic acid.

Compounds of Formula (I) in which R⁶ and R⁷ are each C₁₋₆alkyloxy, represented by Formula (I-e), can be prepared following the synthetic route shown below (Scheme 4) in which R^{6b} and R^{7b} each represents a C₁₋₆alkyloxy group:

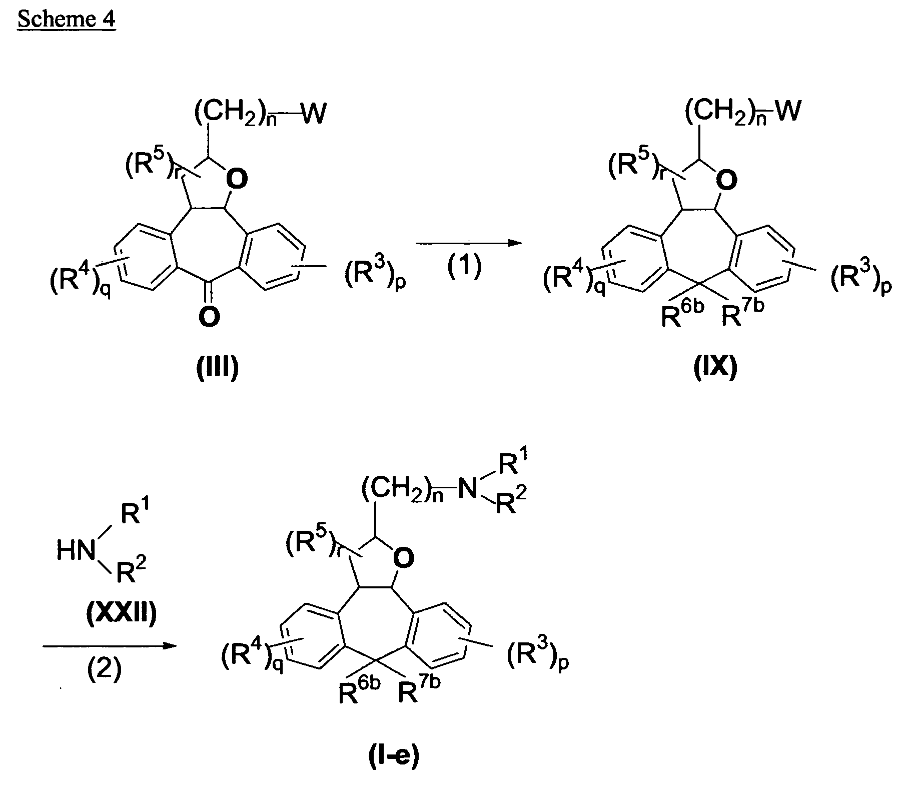

For example, those compounds of Formula (I-e) in which R^{6b} and R^{7b} each represents a methoxy group can be prepared by reacting the compound of Formula (III) with dimethoxysulphone in methanol and hydrochloric acid, in accordance with the procedure described by Tochter et al, Justus Liebigs Annalen der Chemie (1967), 705, 169-84. Other compounds of Formula (I-e) can be prepared by reacting the compound of Formula (III) with the appropriate alkanol in the presence of an appropriate acid catalyst such as camphosulphonic acid or p-toluenesulphonic acid under reflux and azeotropic distillation. The compounds of Formula (IX) can be converted into the final compounds of Formula (I-e) in an analogous manner to that described in Scheme 1, step 5 for the conversion of compounds of Formula (VII) into compounds of Formula (I-a).

Compounds of Formula (I) in which R⁶ and R⁷ are each methyl, represented by Formula (I-f), can be prepared following the synthetic route shown below (Scheme 5) in which R^{6c} and R^{7c} each represents a methyl group:

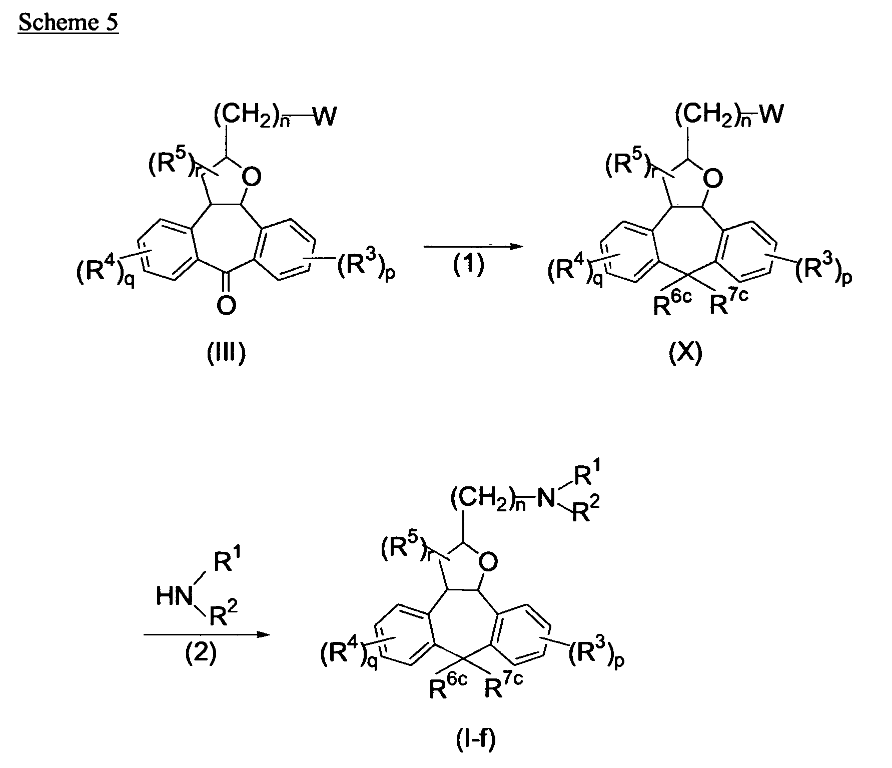

Compounds of Formula (I-f) can be prepared by reacting the compound of Formula (III) with dimethyltitanium dichloride in dichloromethane, in accordance with the procedure described by Reetz et al, Chemische Berichte (1985), 118(3), 1050-7. The compounds of Formula (X) can be converted into the final compounds of Formula (I-f) in an analogous manner to that described in Scheme 1, step 5 for the conversion of compounds of Formula (VII) into compounds of Formula (I-a).

The compounds of Formula (I) in which R⁶ is a C₃₋₆ alkyl group and R⁷ is a C₁₋₆alkyl group can be prepared in an analogous manner from the corresponding compounds of Formula (X).

The compounds of Formula (I) may also be converted into each other following art-known transformation reactions. For instance,
a) a compound of Formula (I), wherein R¹ and R² taken together with the nitrogen atom to which they are attached form a radical of Formula (b), may be converted into the corresponding primary amine by treatment with hydrazine or aqueous alkali;
b) a compound of Formula (I), wherein R¹ or R² is trifluoromethylcarbonyl, may be converted into the corresponding primary or secondary amine by hydrolysis with aqueous alkali;
c) a compound of Formula (I), wherein R¹ or R² is C₁₋₆alkyl substituted with C₁₋₆alkylcarbonyloxy may be hydrolyzed into a compound of Formula (I) wherein R¹ or R² is C₁₋₆alkyl substituted with hydroxy;
d) a compound of Formula (I), wherein R¹ and R² are both hydrogen may be mono- or di-*N*-alkylated to the corresponding amine form;
e) a compound of Formula (I), wherein R¹ and R² are both hydrogen may be *N*-acylated to the corresponding amide;
f) a compound of Formula (I), containing a C₁₋₆alkyloxycarbonyl group may be hydrolyzed to the corresponding carboxylic acid;
g) a compound of Formula (I) in which R³ or R⁴ is fluoro may be treated with an appropriate reagent R^{3a}M or R^{4a}M where M is hydrogen or a metal for example sodium and R^{3a} and R^{4a} each has the meaning for respectively R³ and R⁴ other than fluoro.

The intermediates mentioned hereinabove are either commercially available or may be made following art-known procedures. For instance, intermediates of Formula (II) may be prepared according to the procedure described by Monkovic et al. (J. Med. Chem. (1973), 16(4), p. 403-407).

Intermediates of Formula (II) wherein n is 1 and r is 0, said intermediates being represented by Formula (II-a), can also be prepared by reacting an epoxide derivative of Formula (XI) with a Grignard reagent of Formula (XII) wherein X suitably is halo, thus forming an intermediate of Formula (XIII) which may subsequently be cyclized according to art-known methods such as the one described in Monkovic et al. (J. Med. Chem. (1973), 16(4), p. 403-407).

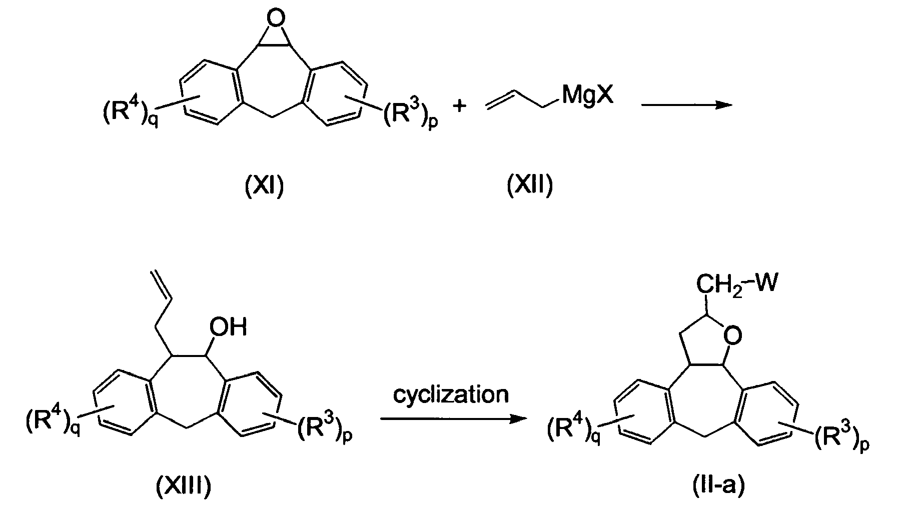

Epoxides of Formula (XI) can be prepared using art-known procedures such as epoxydating an intermediate of Formula (XIV) with a suitable peroxide such as m-chloroperbenzoic acid.

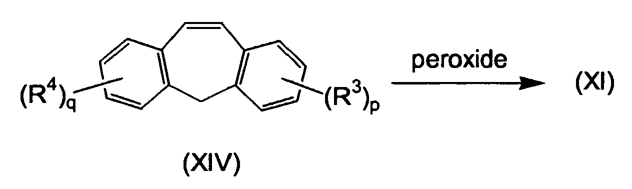

Intermediates of Formula (VI) wherein n is 1 and r is 0 can also be prepared by cyclising an intermediate of Formula (XV) according to art-known methods such as the one described in Monkovic et al. (J. Med. Chem. (1973), 16(4), p. 403-407).

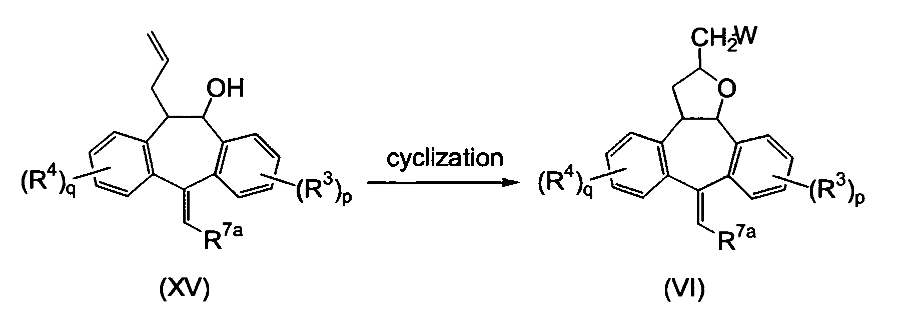

Compounds of Formula (X) above in which R^{6c} is a C₃₋₆ alkyl group and R^{7c} is a C₁₋₆alkyl group can be prepared in accordance with the reaction scheme shown below in which R^{x} is a C₁₋₆alkyl group and R^{6d} is a C₁₋₄alkyl group: 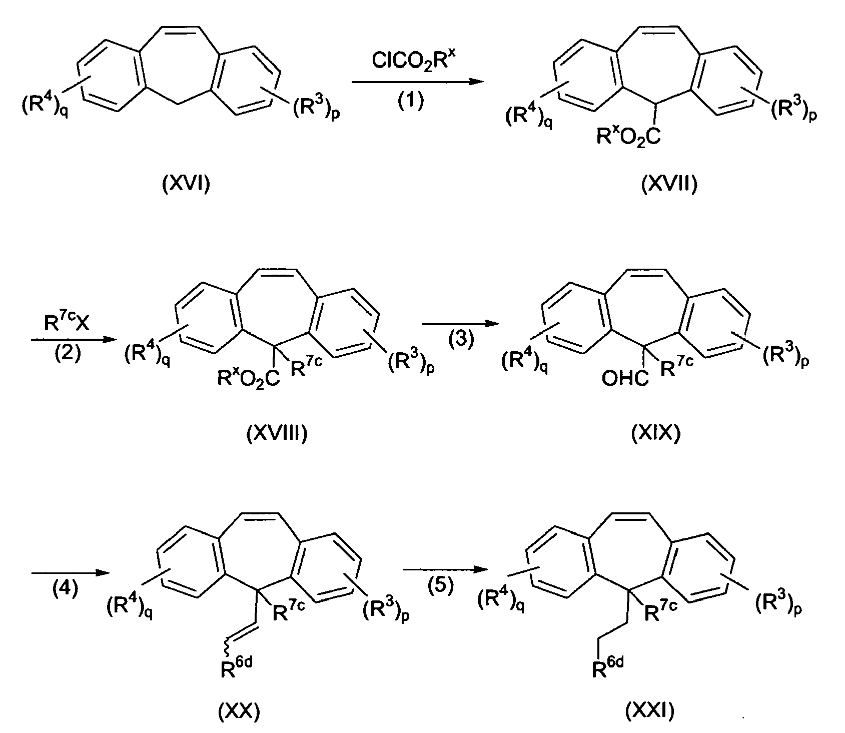
Step 1: a compound of Formula (XVI) is treated with an alkyllithium compound, in an inert reaction solvent as for example tetrahydrofuran, and a compound of Formula ClCO₂R^{x} to form a compound of Formula (XVII);
Step 2: a compound of Formula (XVII) is reacted with an alkali metal (e.g. potassium) C₁₋₆alkoxide, in an inert reaction solvent such as for example tetrahydrofuran, and with a compound of Formula R^{7c}-X where X is for example iodo to form a compound of Formula (XVIII);
Step 3: a compound of Formula (XVIII) is treated with Di-isobutylaluminium hydride (DIBAL-H) to form a compound of Formula (XIX);
Step 4: a compound of Formula (XIX) is subjected to a Wittig type reaction to form a compound of Formula (XX);
Step 5: a compound of Formula (XX) is hydrogenated for example with platinum or palladium on carbon in an organic solvent such as for example, methanol, isopropyl alcohol, acetone or ethyl acetate and under hydrogen pressure, as for example atmospheric pressure, and room temperature to form a compound of Formula (XXI).
The compound of Formula (XXI) can then be epoxydated and cyclized in accordance with the procedures described above to form a compound of Formula (X).

The following examples are intended to illustrate and not to limit the scope of the present invention.

### Experimental part

### A. Preparation of the intermediate compounds

Hereinafter, "DCM" is defined as dichloromethane, "DIPE" is defined as diisopropyl ethyl ether, IPA is define as isopropyl alcohol, "DMF" is defined as *N,N*-dimethylformamide, "EtOAc" is defined as ethyl acetate, "EtOH" is defined as ethanol, "MeOH" is defined as methanol "THF" is defined as tetrahydrofuran, "TFA" is defined as trifluoroacetic acid.

### A. Preparation of the intermediate compounds

### Example A1

a) Preparation of intermediate compound 1 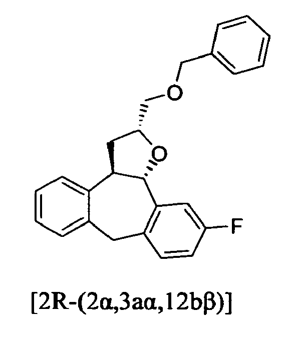 [2R-(2α,3aα,12bβ)]-11-fluoro-2-hydroxymethyl-3,3a,8,12b-tetrahydro-2*H-*dibenzo[3,4:6,7]cyclohepta[1,2-b]furan(0.0757 mol), sodium hydride 60 % in mineral oil (0.08327 mol), (bromomethyl)- benzene (0.1514 mol) and THF dry (300 ml) were mixed at room temperature and then heated for 3 hours at reflux temperature. After cooling to room temperature, water was added and layers were separated. The organic phase was dried (Na₂SO₄) and volatiles were evaporated under vacuum. The residue thus obtained was purified by short open column chromatography. The product fractions were collected and the solvent was evaporated, yielding quantitatively intermediate compound 1.
b) Preparation of intermediate compound 2 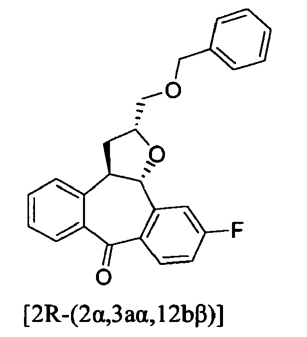 To solution of intermediate compound 1 (0.15234 mol) in DCM (1500 ml) stirred at room temperature, a solution of potassium permanganate (0.27421 mol), potassium hydroxide (0.1112 mol) and Bu₄N⁽⁺⁾HSO₄⁽⁻⁾ (0.02285 mol) in water (1500 ml) was added, then the reaction mixture was stirred for 16 hours at room temperature and then acidified with 1 N HCl. Small portions of NaHSO₃ were added until colour changed from brown to pale yellow. The bottom layer was separated, washed with brine and with water, then dried over Na₂SO₄ and the solvent was evaporated. The remaining oily residue was purified by short open column chromatography (eluent: EtOAc/Heptane 1/9). The product fractions were collected and the solvent was evaporated, yielding intermediate compound 2.
c) Preparation of intermediate compound 3 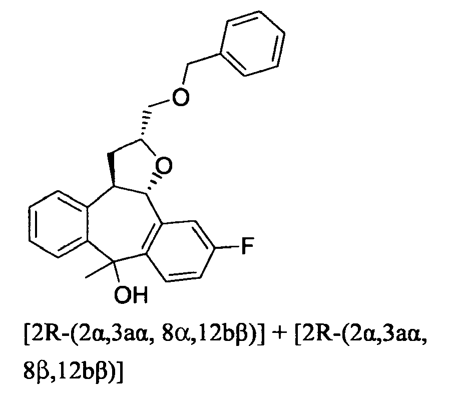 The following reaction was conducted under N₂ atmosphere: methyl magnesium chloride 3M in THF (0.090 mol) was added via a syringe to a solution of intermediate compound 2 (0.04590 mol) in THF (350 ml) at room temperature.The reaction mixture was further stirred for 6 hours at room temperature. A saturated aqueous NH₄Cl solution was added and the organic solvent was evaporated under vacuum. The obtained concentrate was taken up in DCM, washed with brine and with water, then dried (Na₂SO₄) and concentrated (vacuum), yielding intermediate compound 3 as a mixture of diastereoisomers at position 8 (used as such in the next reaction step without further purification).
d) Preparation of intermediate compound 4 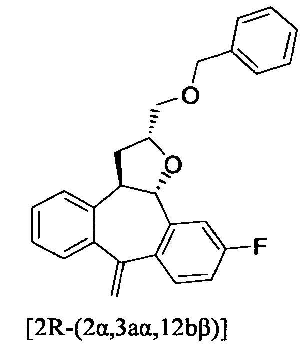 Thionyl chloride (0.13350 mol) was added dropwise to a stirred solution of intermediate compound 3 (0.04450 mol) in pyridine (170 ml) at 0 °C. After reaching room temperature, the reaction mixture was further stirred overnight and then the volatiles were evaporated under vacuum. The residue thus obtained was taken up in DCM and washed with a saturated aqueous NaHCO₃ solution, with brine and with water. The separated organic layer was dried (Na₂SO₄) and the solvent was evaporated (vac.), yielding intermediate compound 4 (used in the next reaction step without further purification).
e) Preparation of intermediate compound 5 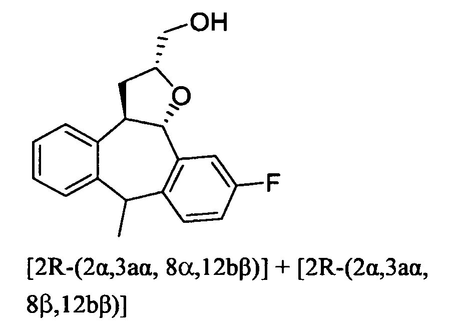 A mixture of intermediate compound 4 (0.044 mol), H₂ (1 atm.) and Pd/C 10 % (cat. quant.) in MeOH/EtOAc (100 ml) was shaken for 24 hours at 1 atm., then the catalyst was removed, a new catalytic portion of Pd/C 10% was added and the mixture was shaken for an additional 12 hours under H₂ atmosphere (30 psi). The catalyst was filtered off and the solvent was evaporated. The residue thus obtained was purified by short open column chromatography. The product fractions were collected and the solvent was evaporated, yielding intermediate compound 5 (mixture of diastereoisomers at position 8).
f) Preparation of intermediate compound 6 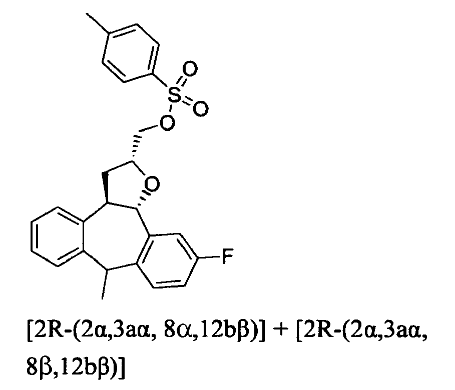 A mixture of intermediate compound 5 (0.04022 mol), triethylamine (0.16088 mol) and 4-methyl-benzenesulfonyl chloride (0.08044 mol) in DCM dry (300 ml) was stirred under N₂ atmosphere for 12 hours at room temperature, then water was added and the layers were separated. The organic layer was dried (Na₂SO₄), the solid was filtered off and the solvent was evaporated (vac.). The residue thus obtained was purified by short open column chromatography. The product fractions were collected and the solvent was evaporated, yielding intermediate compound 6 (mixture of diastereoisomers at position 8).

### Example A2

a) Preparation of intermediate compound 7 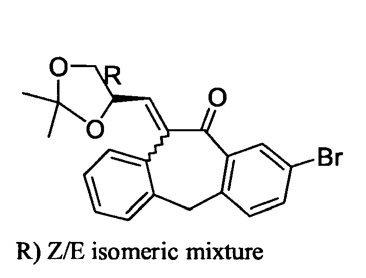 The following reaction was conducted under N₂ atmosphere: Magnesium chloride (0.3342 mol) was added to a solution of 8-bromo-5,11-dihydro-10*H*-dibenzo[a,d]-cyclohepten-10-one (0.2786 mol) in THF dry (1600 ml) and the reaction mixture was stirred at room temperature for 0.5 hour. R-2,2-dimethyl-1,3-dioxolane-4-carboxaldehyde (0.4736 mol) was added dropwise over 5 min. (via funnel), then potassium tert-butoxide (0.05566 mol) was added in one portion and the mixture was stirred at room temperature for 16 hours. Water (320 ml) was added slowly, the mixture was stirred for 10 min. (slightly exothermic reaction) and then HCl (80 ml, 2 N) was added. The mixture was stirred for 15 min. and was extracted with DCM. The organic layer was dried (Na₂SO₄) and the solvent was evaporated. The residue was purified by short open column chromatography (eluent: DCM). The product fractions were collected and the solvent was evaporated, yielding 103.28 g (93 %) of intermediate compound 7 as a mixture of Z/E isomers.
b) Preparation of intermediate compounds 8 and 9 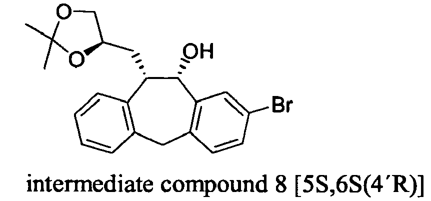 and 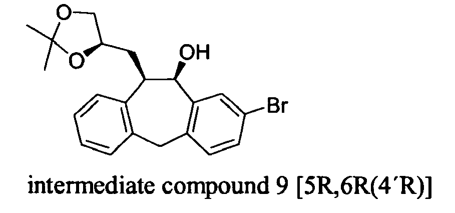 Sodium borohydride (0.5173 mol) was added portionwise to a solution of intermediate compound 7 (0.2587 mol) in EtOH absolute (4000 ml) and THF dry (600 ml) and the reaction mixture was stirred at room temperature for 2 days. A 10 % NH₄Cl aqueous solution (500 ml)was added slowly, then water (500 mL) was added, then the organic solvent was evaporated (under vacuum) and the concentrate was extracted with DCM. The organic layer was dried (Na₂SO₄) and the solvent was evaporated. The residue was purified by short open column chromatography (eluent: DCM/MeOH 98/2), then purified by high-performance liquid chromatography (eluent: DCM/MeOH 98/2). Two product fractions were collected and for each solvents were evaporated, yielding 29 g of intermediate compound 8 and 32 g of intermediate compound 9.
c) Preparation of intermediate compound 10 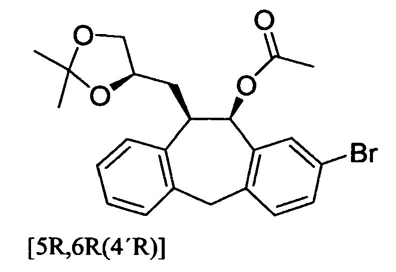 Acetic acid, anhydride (0.0775 mol) was added to a solution of intermediate compound 9 (0.0705 mol), triethylamine (0.0774 mol) and *4-N,N*-dimethyl-aminopyridine (0.0035 mol) in dry DCM (400 ml) and the reaction mixture was stirred at room temperature for 16 hours, then the mixture was washed with a 10 % NH₄Cl solution. The organic layer was dried and the solvent was evaporated. The residue was purified by short open column chromatography (eluent: DCM/MeOH 100/0, 98/2). The product fractions were collected and the solvent was evaporated, yielding 32.5 g (94 %) of intermediate compound 10.
d) Preparation of intermediate compound 11 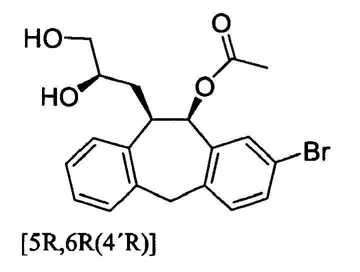 A mixture of intermediate compound 10 (0.0802 mol) in acetic acid 64 ml) and water (40 ml) was stirred at 55 °C for 8 hours, then water (200 ml) was added and the reaction mixture was extracted 3 times with DCM (3x 250 ml). The organic layer was dried (Na₂SO₄) and the solvent was evaporated. The residue was purified by short open column chromatography (eluent: DCM/MeOH 100/0, 98/2, 96/4). The product fractions were collected and the solvent was evaporated, yielding 31.34 g of intermediate compound 11.
e) Preparation of intermediate compound 12 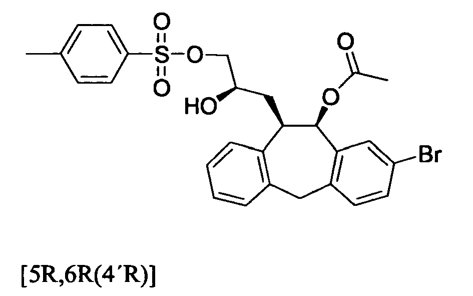 A mixture of intermediate compound 11 (0.0730 mol) and triethylamine (0.0803 mol) in DCM dry (800 ml) was cooled on an ice-bath. Dibutyloxotin (0.0073 mol), then 4-methyl-benzenesulfonyl chloride (0.1095 mol) were added and the reaction mixture was allowed to reach room temperature. The mixture was stirred at room temperature for 16 hours. 10 % aqueous NH₄Cl solution was added. The organic layer was separated, dried (Na₂SO₄) and the solvent was evaporated, yielding 40.84 g of intermediate compound 12.
f) Preparation of intermediate compound 13 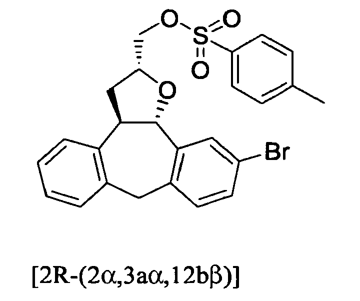 Methanesulfonic acid (0.1241 mol) was added to a solution of intermediate compound 12 (0.0730 mol) in toluene (400 ml) and the reaction mixture was stirred at room temperature for 2 days, then washed with sodium carbonate (1M). The organic layer was dried and the solvent was evaporated. The residue was purified by short open column chromatography (eluent: DCM), yielding 15.27 g (42 %) of intermediate compound 13.
g) Preparation of intermediate compound 14 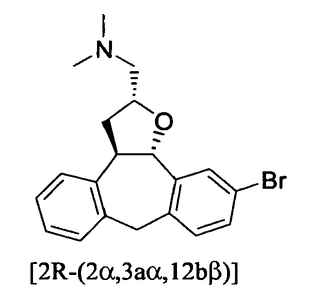 A mixture of intermediate compound 13 (0.0305 mol), *N,N*-dimethylamine (0.820 mol) and calcium oxide (0.421 mol) in THF dry (500 ml) was stirred at 140 °C (oil bath temperature) for 16 hour into a pressurized vessel. After cooling to rt the solid residues were filtered off and the filtrate was evaporated until dryness. The residue thus obtained was purified by short open column chromatography (eluent: DCM/(MeOH/NH₃) 98/2). The product fractions were collected and the solvent was evaporated, yielding 8.10 g of intermediate compound 14.
h) Preparation of intermediate compound 15 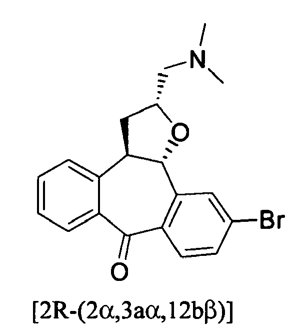 Chromium (VI) oxide (0.04405 mol) was added to a solution of intermediate compound 14 (0.01101 mol) in acetic acid (100 ml), then the reaction mixture was stirred and refluxed for 3 hours. After cooling to room temperature, the solvent was evaporated (vac.) and the resulting residue was taken up in EtOAc and water. A saturated aqueous NH₄Cl solution was carefully added until the gas-evolution stopped. The organic layer was separated, washed with brine and with water, then dried (Na₂SO₄), filtered off and the solvent was evaporated (vac.). The residue thus obtained was purified by short open column chromatography over silica gel (eluent: DCM/(MeOH/NH₃) mixtures). The desired product fractions were collected and the solvent was evaporated, yielding 2.1 g of intermediate compound 15.
j) Preparation of intermediate compound 16 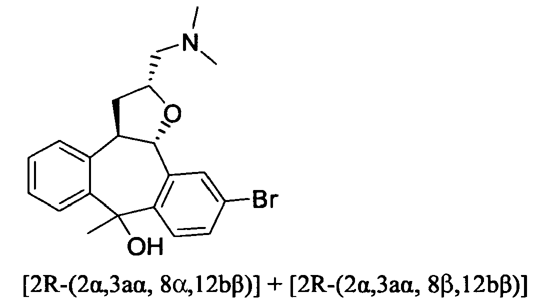 The following reaction was conducted under N₂ atmosphere: methylmagnesium chloride 3M in THF (0.015 mol) was added at room temperature to a solution of intermediate compound 15 (0.00543 mol) in THF dry (40 ml). The reaction mixture was stirred at room temperature for 16 h and then water was carefully added and the layers were separated. The organic layer was dried (Na₂SO₄), filtered off and the solvent was evaporated (vac.). The residue was purified by short open column chromatography (eluent: DCM/(MeOH/NH₃)). The product fractions were collected and the solvent was evaporated, yielding intermediate compound 16 (mixture of diastereoisomers).

### Example A3

a) Preparation of intermediate compound 17 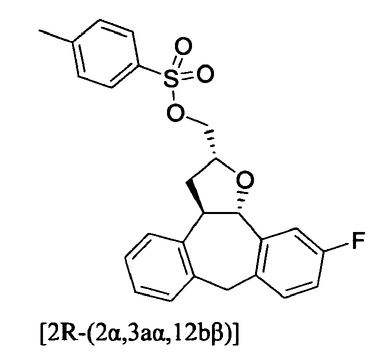 [2R-(2α,3aα,12bβ)]-11-fluoro-2-hydroxymethyl-3,3a,8,12b-tetrahydro-2*H*-dibenzo-[3,4:6,7]cyclohepta[1,2-b]furan (4.1 g, 0.0144 mol) was dissolved in DCM (20 ml). Triethylamine (0.0173 mol) was added and the mixture was cooled on an ice-bath. A solution of 4-methyl-benzenesulfonyl chloride (0.0159 mol) in DCM (10 ml) was added over ± 5 min. The mixture was stirred for 2 hours at 0°C, then allowed to warm to room temperature. The reaction mixture was stirred overnight at room temperature. Water (20 ml) was added. The layers were separated. The organic phase was stirred in a 10 % aqueous potassium carbonate solution for one hour. The layers were separated. The organic layer was dried (MgSO₄), filtered and the solvent evaporated. The residue was stirred in toluene (30 ml). A 10% aqueous potassium carbonate solution (30 ml) was added. The layers were separated. The organic layer was dried (MgSO₄), filtered and the solvent evaporated, yielding 5.8 g (92%) of intermediate compound 17.
b) Preparation of intermediate compound 18 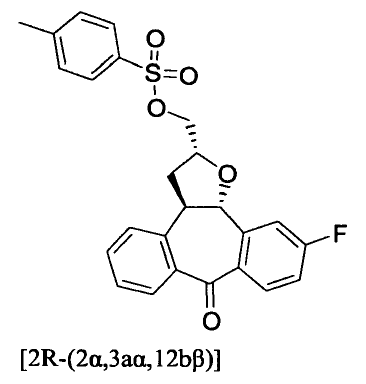 Intermediate compound 17 (60 g, 137 mmol) was dissolved in DCM (600 ml) and stirred at room temperature for 15 min. Then, a solution of potassium permanganate (37.76 g, 233 mmol), potassium hydroxide (4.22 g, 75 mmol) and tetrabutylammonium hydrosulfate (5.58 g, 16 mmol) in water (600 ml) was added. The mixture was stirred at room temperature for 16 hour. Then, the dark brown mixture was acidified with HCl 1N and small portions of NaHSO₃ were added until the brown color disappeared. The bottom layer was separated, washed with brine, water, dried (Na₂SO₄) and evaporated under vacuum. The resulting colorless oil was purified by short open column chromatography (silica gel, eluent: EtOAc/Heptane 1:9) to give 48.29 g of intermediate compound 18.
c) Preparation of intermediate compound 19 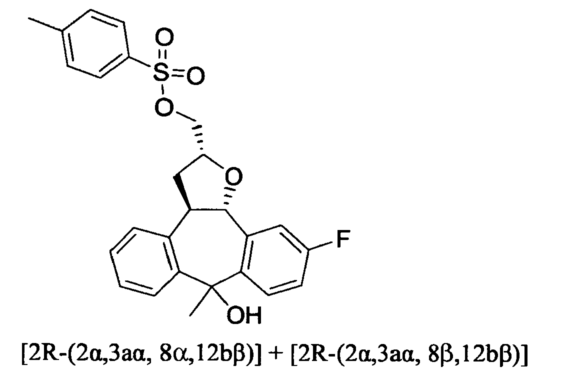 To intermediate compound 18 (48.28 g, 107 mmol) in dry THF (1000 mL) stirred at room temperature, chloromethyl-magnesium (107 ml of a solution 3 M in THF, 321 mmol) was added dropwise under a N₂ atmosphere. The reaction mixture was stirred at room temperature for 5 hours. Then, NH₄Cl (100 ml of aqueous saturated solution) was carefully added and the resulting mixture was stirred for 15 min at room temperature. DCM was added (1000 mL) and the organic layer was separated, dried (Na₂SO₄) and evaporated under vacuum to give a residue that was triturated with diethyl ether. The white precipitate formed was filtered off, washed with cold diethyl ether (3 x 50 ml) and dried under vacuum to give 33.75 g of intermediate compound 19 as a 1:1 diastereoisomeric mixture.
d) Preparation of intermediate compound 20 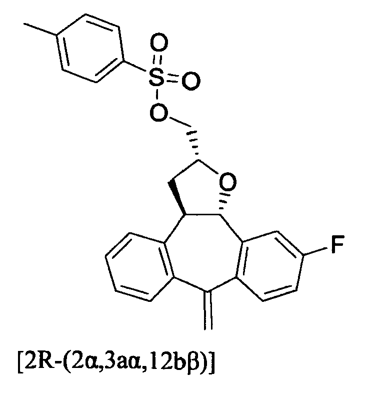 To a solution of intermediate compound 19 (49.52 g, 106 mmol) in pyridine (200 mL), stirred at -5 °C under N₂ atmosphere, thionyl chloride (19.27 ml, 264 mmol) was dropwise added. The resulting mixture was gradually warmed to room temperature and stirred for 16 hours. The volatiles were evaporated under vacuum and the resulting residue was taken up with DCM (200 ml) and sequentially washed with NaHCO₃ (2 x 100 ml of aqueous saturated solution), HCl 1 N (2 x 100 ml) and brine (100 ml). The washed organic layer was dried (Na₂SO₄) and evaporated under vacuum. The residue thus obtained was purified by short open column chromatography (silica gel, eluent DCM/heptane 1:1) to give 40 g of intermediate compound 20 as a white syrup.
e) Preparation of intermediate compound 21 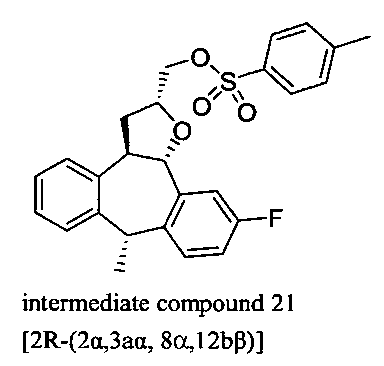 To a suspension of Pd/C 10 % (0.05 equiv.) in acetone (200 mL) under N₂ atmosphere, a solution of intermediate compound 20 (40 g, 88.78 mmol) in acetone (200 ml) was added at room temperature. The flask was evacuated and filled with hydrogen until the pressure reached 20 psi. The resulting suspension was shaken at room temperature for 16 hours. The catalyst was filtered off and the filtrate was evaporated under vacuum to give 40 g of a mixture of intermediate compound 6 as a mixture of diastereoisomers and intermediate compound 21 ; the latter was isolated from the mixture by precipitation with diethyl ether to give intermediate compound 21 (17 g) as a white solid.
f) Preparation of intermediate compound 22 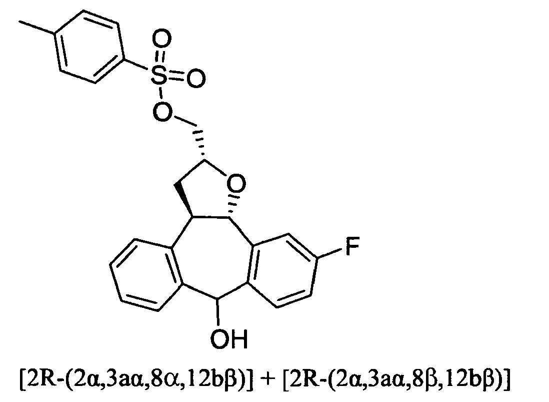 Sodium borohydride (5 mmol) was added portionwise to a solution of intermediate compound 18 (2.5 mmol) in MeOH (15 ml) and THF dry (30 ml) and the reaction mixture was stirred at room temperature for 4 hours. A NH₄Cl aqueous saturated solution (50 ml) was added slowly was added and the resulting mixture was stirred for 15 min. at room temperature. The volatiles were evaporated (under vacuum) and the concentrate was taken up in DCM and washed with brine and water. The organic layer was separated, dried (Na₂SO₄) and the solvent was evaporated. The residue was purified by short open column chromatography (eluent: DCM/MeOH 98/2), yielding 0.9 g of intermediate compound 22 as a mixture of diastereoisomers.
g) Preparation of intermediate compound 23 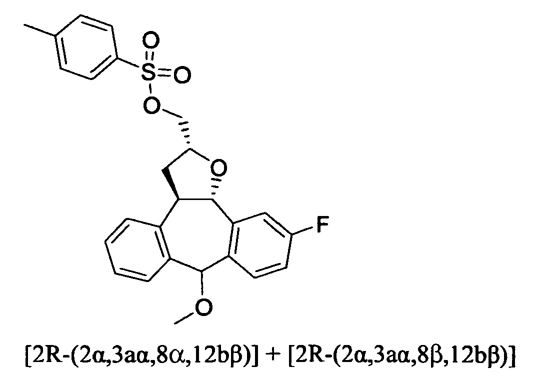 To a solution of intermediate compound 22 (1.92 mmol) in THF (50 ml) at room temperature, sodium hydride (2.5 mmol) was portionwise added. The mixture was stirred at room temperature for 30 minutes. Methyliodine (12.5 mmol) was added and the resulting mixture was refluxed for 6 hours. After cooling to room temperature, water was added and volatiles were evaporated under vacuum. DCM was added and layers were separated. The organice layers were washed with brine, dried (Na₂SO₄), filtered off and vacuum concentrated affording a residue that was purified by short open column chromatography affording 0.3 g of intermediate compound 23 as a mixture of diastereoisomers.

### Example A4

a) Preparation of intermediate compound 24 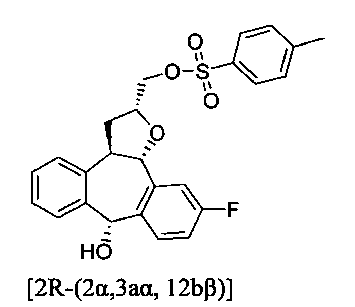 A mixture of intermediate compound 18 (0.0066 mol) and sodium tetrahydroborate (0.033 mol) in EtOH (100 ml) was stirred for 3 hours at room temperature (if necessary, THF (20 ml) was added to improve the solubility) and then the solvent was evaporated under reduced pressure. The residue was dissolved in DCM and the solution was washed with water and with brine. The organic layer was dried (Na₂SO₄), filtered off and the solvent was evaporated. The residual oil was purified by short open column chromatography (eluent: DCM). The pure fractions were collected and the solvent was evaporated, yielding 2.2 g of intermediate compound 24.
b) Preparation of intermediate compound 25 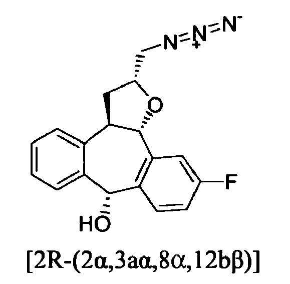 A mixture of intermediate compound 24 (0.00055 mol) and sodiumazide (0.001155 mol) in DMF (10 ml) was stirred for 16 hours at 85°C and then the reaction mixture was partitioned between water and DCM. The organic layer was washed with water and with brine, dried (Na₂SO₄), filtered off and the solvent was evaporated. The residual oil was purified by short open column chromatography (eluent: DCM/(MeOH/NH₃) 95/5). The product fractions were collected and the solvent was evaporated, yielding 0.15 g (84 %) of intermediate compound 25.

### B. Preparation of the final compounds

### Example B1

### Preparation of final compound 41

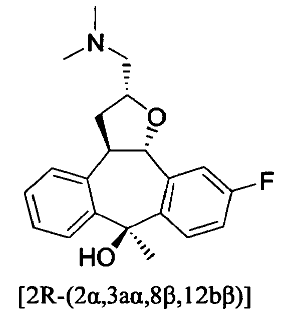

Reaction conducted under N₂ atmosphere: a solution of final compound 13 (0.00325 mol) in THF (50 ml) was cooled to -30 °C. Then, methylmagnesium chloride (0.0190 mol) was added dropwise and the resulting reaction mixture was stirred for 60 min at room temperature (cooling bath was removed).The reaction mixture was stirred at room temperature for 8 hours. The reaction mixture was treated with a saturated aqueous NH₄Cl solution, then extracted two times with DCM. The combined organic layers were washed with brine, dried, filtered, and the solvent was evaporated. The residue was purified (eluent: DCM/(MeOH/NH₃) 95/5). The pure fractions were collected and the solvent was evaporated, yielding final compound 41.

### Example B2

### Preparation of final compound 26

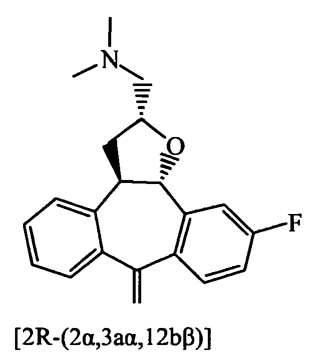

A solution of final compound 41 (0.00081 mol) in pyridine (6 ml) was stirred at 0°C. Thionyl chloride (1.2 ml) was dropwise added. The reaction mixture was stirred for 25 minutes at 0 °C, then poured out onto ice and this mixture was extracted twice with DCM. The combined organic layers were washed with brine, dried (Na₂SO₄),, filtered and the solvent evaporated in vacuo, yielding 0.200 g of final compound 26.

### Example B3

### Preparation of final compounds 64 and 66

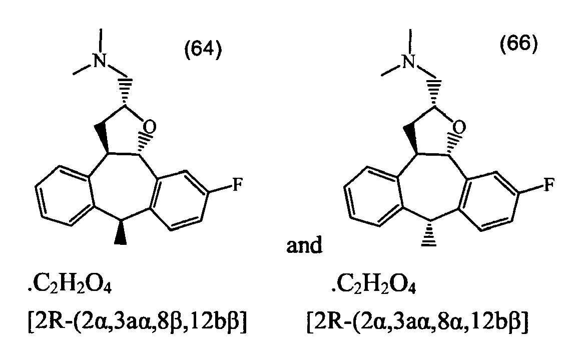

To a suspension of Pd/C 10 % (catalytic quantity) in EtOH (100 ml), final compound 26 (0.00136 mol) was added at room temperature. This mixture was hydrogenated for 6 hours under H₂ pressure. After uptake of H₂ (1 equiv), the catalyst was filtered off and the filtrate was evaporated in vacuo. The residue was purified by Circular chromatography (eluent: DCM/MeOH from 100/0 to 95/5). Two diastereomer fraction groups were collected and their solvent was evaporated. Each residue was dissolved in Et₂O dried and a solution of oxanic acid (1.2 equivalents) in Et₂O was added. The resulting mixture was stirred for 15 min and the white precipitates obtained were filtered off and washed with dry cold Et₂O affording each corresponding ethanedioic acid salts (1:1) 0.029 g final compound 64 and 0.19 g final compound 66.

### Example B4

### Preparation of final compounds 65, 67 and 68

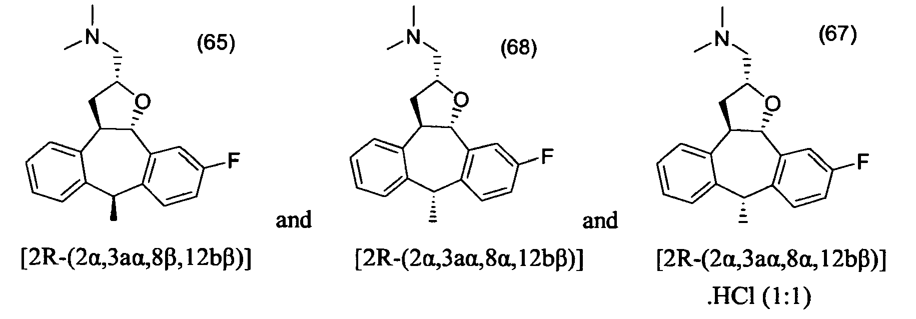

A mixture of intermediate compound 6 (0.01546 mol), *N,N*-dimethylamine (0.030 mol) and calcium oxide (1 g) in THF (100 ml) was heated for 8 hours in a parr pressurised reactor vessel at 130 °C (oil bath temperature). After cooling to room temperature, the solids were filtered off and the solvent was evaporated. The obtained residue was taken up in DCM and then washed with a saturated aqueous sodium carbonate solution, with brine and with water. The organic layer was separated, dried (Na₂SO₄), filtered off and the solvent was evaporated (under vacuum.). The resulting residue was purified by flash column chromatography. Two product fractions were collected and the solvent was evaporated, yielding final compound 68 and final compound 65 . A fraction of final compound 68 was taken up in diethyl ether (100 ml) and MeOH (20 ml) and converted into the corresponding hydrochloride salt by addition of i-PrOH/HCl (saturated solution, 15 ml). The white precipitate thus obtained was filtered off and washed with cold dry diethyl ether (3 x 100 ml) to give final compound 67.

### Alternative Preparation of final compounds 67 and 68:

To a solution of intermediate compound 21 (17 g, 37.56 mmol) in THF (200 ml), calcium oxide (14.64 g, 375.6 mmol) and *N,N-*dimethylamine 1 M in THF (375 ml, 375 mmol) were added at room temperature. The resulting mixture was heated at 130 °C (oil bath temperature) into a Parr pressure vessel for 8 hours. After cooling the reaction to room temperature, the solid was filtered off and the filtrate was evaporated under vacuum. The residue thus obtained was taken up with DCM (100 ml) and washed with NaHCO₃ (aqueous saturated solution, 2 x 100 ml), brine (2 x 100 ml) and water (100 ml). The organic layer was dried (Na₂SO₄) and vacuum concentrated affording a residue that was purified by short open column chromatography (silica gel, eluent. DCM/MeOH(NH₃)sat 2.5 %) to give 10.5 g of final compound 68 as a colorless oil. Final compound 68 was taken up in diethyl ether (100 ml) and MeOH (20 ml) and converted into the corresponding hydrochloride salt by addition of ⁱPrOH/HCl (saturated solution, 15 ml). The white precipitate was filtered off and washed with cold dry diethyl ether (3 x 100 ml) to give 11.05 g of final compound 67 as a white solid.

### Example B5

### Preparation of final compound 20

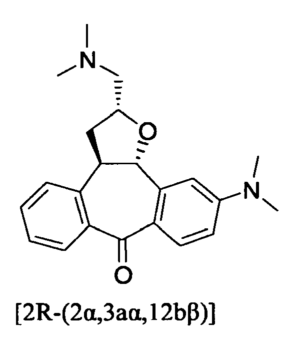

To a solution of intermediate compound 18 (6.25 g, 13.81 mmol) in THF (50 ml), calcium oxide (5.38 g, 138 mmol) and *N,N*-dimethylamine 1 M in THF (138 ml, 138 mmol) were added at room temperature. The resulting mixture was heated at 140 °C (oil bath temperature) into a Parr pressure vessel for 16 hours. After cooling the reaction to room temperature, the solid was filtered off and the filtrate was evaporated under vacuum. The residue thus obtained was taken up with DCM (100 ml) and washed with NaHCO₃ (aqueous saturated solution, 2 x 100 ml), brine (2 x 100 ml) and water (100 ml). The organic layer was dried (Na₂SO₄) and vacuum concentrated affording a residue that was purified by short open column chromatography (silica gel, eluent. DCM/MeOH(NH₃)sat 2.5 %) to give 0.290 g of final compound 20 as a colorless oil..

### Example B6

a) Preparation of final compound 13 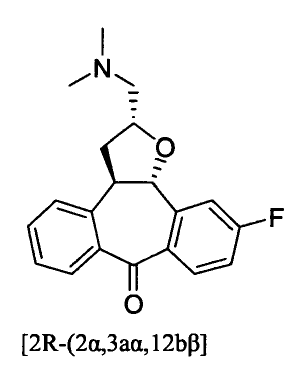 Chromium(VI)oxide (0.1076 mol) was added in small portions to a solution of 11-fluoro-3,3a,8,12b-tetrahydro-*N,N*-dimethyl-2*H*-dibenzo[3,4:6,7]cyclohepta[1,2-b]furan-2-methanamine [2R-(2α,3aα,12bβ)] (0.0269 mol) in acetic acid- (200 ml), then the reaction mixture was stirred and refluxed for 6 hours. After cooling to room temperature, the solvent was evaporated and the obtained residue was taken up in EtOAc/NaHCO₃ (aqueous saturated solution). The organic layer was separated and the aqueous layer was extracted 2 times with EtOAc. The organic layers were combined, washed with brine, dried (Na₂SO₄), filtered off and the solvent was evaporated. The residue thus obtained was purified by short open column chromatography, then the product fractions were collected and the solvent was evaporated, yielding 3.56 g (40.6 %) of final compound 13.
b) Preparation of final compound 15 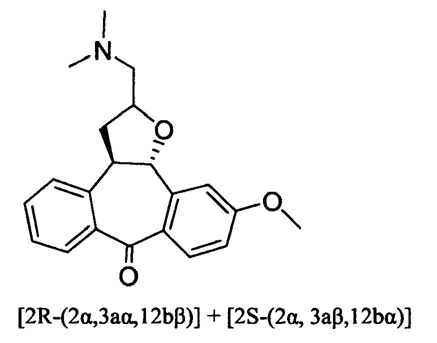 A mixture of final compound 13 (0.0045 mol) in sodium methoxyde 33 % in MeOH (1.46 ml), MeOH (15 ml) and dioxane (10 ml) was heated for 16 hours at 130 °C (oil bath temperature) into a Parr pressure reactor vessel. After cooling to room temperature the solvent was evaporated. The resulting residue was dissolved in DCM, washed with water and with brine, then dried (Na₂SO₄) and the solvent was evaporated. The residue was purified by short open column chromatography, then the product fractions were collected and the solvent was evaporated, yielding 1.04 g of final compound 15 as a mixture of diastereoisomers at position 2..

### Example B7

### Preparation of final compound 18

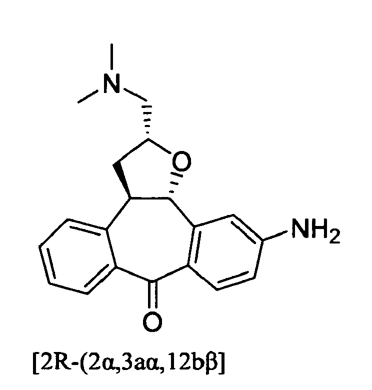

Final compound 13 (0.00372 mol) was dissolved in 1,4-dioxane (50 ml), and NH₄OH 30 % in water (7 ml) was added. The reaction mixture was stirred for 22 hours at 80 °C into a Parr pressure reactor vessel, then stirred for 16 hours at 140 °C. The reaction mixture was cooled to room temperature and water/DCM were added. The organic layer was separated, washed twice with a saturated aqueous NaHCO₃ solution, then with brine, dried (Na₂SO₄), filtered and the solvent was evaporated. The resulting residue was purified by column chromatography. The desired fractions were collected and the solvent was evaporated, yielding 0.460 g of final compound 18.

### Example B8

### Preparation of final compound 14

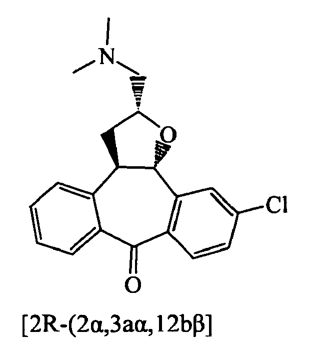

The following reaction was conducted under N₂ atmosphere: 1,1-dimethylethyl nitrite (0.00279 mol) was added dropwise to a solution of copper(II)chloride (0.00223 mol) in acetonitrile dry (q.s.) and after 5 min. Final compound 18 (0.00186 mol) was added portionwise and then the reaction mixture was stirred and refluxed for 1 hour. The mixture was quenched with aqueous 1 N HCl and extracted twice with DCM. The organic extracts were combined, washed with brine, dried (Na₂SO₄), filtered and the solvent was evaporated. The residue was purified by radial chromatography (eluent: DCM/(MeOH/NH₃) 100/0, 98/2). The product fractions were collected and the solvent was evaporated, yielding 0.203 g of final compound 14.

### Example B9

### Preparation of final compound 81

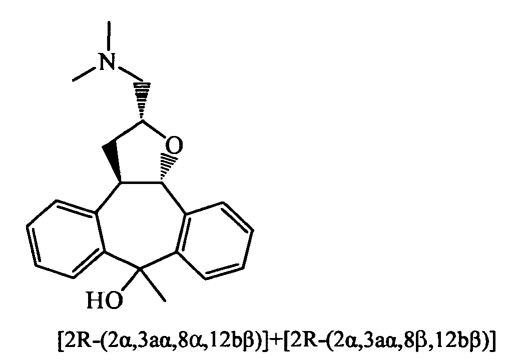

The following reaction was conducted under N₂: A solution of intermediate compound 16 (0.00274 mol) in THF dry (20 ml) was cooled to -20°C and n-butyllithium (0.005 mol) was added dropwise. The reaction mixture was stirred and allowed to reach room temperature overnight. Water was added. The organic layer was separated, washed with brine and with water, then dried (Na₂SO₄), filtered off and the solvent was evaporated under vacuum. The resulting residue was purified by short open column chromatography (eluent: mixtures of DCM/MeOH/NH₃(sat.)). The product fractions were collected and the solvent was evaporated, yielding final compound 81 (mixture of 2 diastereoisomers (2/1)).

### Example B10

a) Preparation of final compound 36 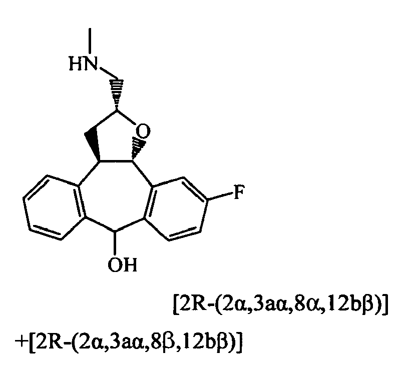 A mixture of final compound 3 (22 mmol) and MeOH (150 ml) was stirred under N₂ atmosphere. Sodium borohydride (53.96 mmol) was added portionwise. The reaction mixture was stirred at room temperature for 1 hour. An additional amount of sodium borohydride (13.5 mmol) was added. The mixture was stirred for 30 minutes at room temperature under N₂ atmosphere. The solvent was evaporated (vacuum ; 40 °C). The residue was stirred in water/DCM. The separated aqueous layer was extracted with DCM (2x). The combined organic layers were washed with water, dried (Na₂SO₄), filtered and the solvent was evaporated (vacuum ; 40°C). This residue (7 g) was purified by column chromatography over silica gel (eluent : DCM/MeOH 95/5). The desired fractions were collected and the solvent was evaporated, yielding 1.064 g of final compound 36 as a mixture of diastereoisomers at position 8.
b) Preparation of final compounds 33 and 34 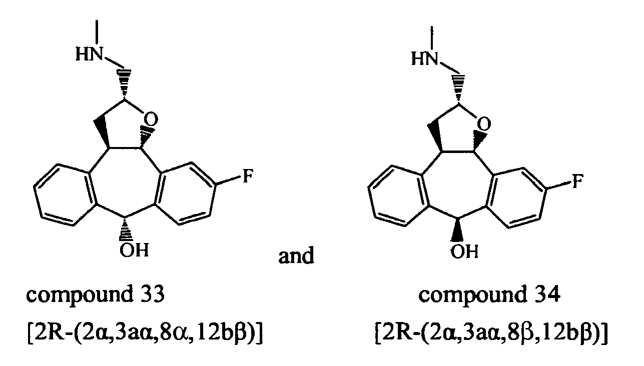 Final compound 36 (0.0028 mol) was dissolved in MeOH (5 ml). This solution was purified by high performance liquid chromatography over Kromasil KR 100-10 (eluent: MeOH/CH₃CN/(NH₄OAc 0.5M pH7.0) 19:19:62). The desired fractions were collected and evaporated, yielding final compound 33 and final compound 34.
c)Preparation of final compound 60 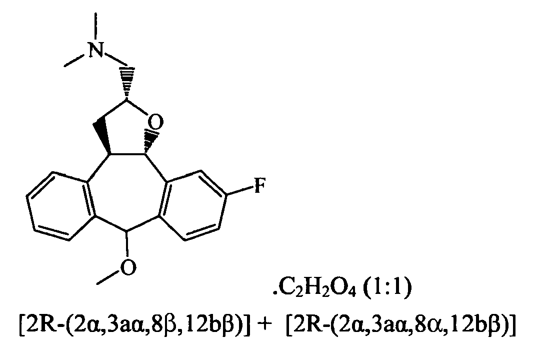 To a solution of intermediate compound 23 (0.3 g, 0.64 mmol) in THF (20 ml), calcium oxide (100 mg, 2.56 mmol) and *N,N-*dimethylamine 2 M in THF (5 ml, 10 mmol) were added at room temperature. The resulting mixture was heated at 130 °C (oil bath temperature) into a Parr pressure vessel for 8 hours. After cooling the reaction to room temperature, the solid was filtered off and the filtrate was evaporated under vacuum. The residue thus obtained was taken up with DCM (100 ml) and washed with NaHCO₃ (aqueous saturated solution, 2 x 100 ml), brine (2 x 100 ml) and water (100 ml). The organic layer was dried (Na₂SO₄) and vacuum concentrated affording a residue that was purified by short open column chromatography (silica gel, eluent. DCM/MeOH(NH₃)sat 2.5 %) to give a residue that was converted into its corresponding oxalate salt by treatment with oxalic acid (1.2 equivalents) in Et₂O at room temperature affording final compound 60 as a white solid.
d) Preparation of final compound 35 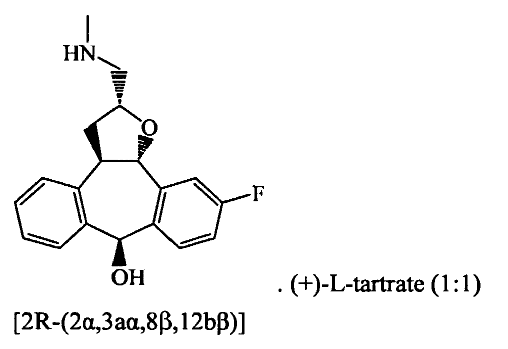 Final compound 34 (0.367 mmol) was dissolved in acetone (2 ml) while heating. (+)-[R-(R*,R*)]-2,3-dihydroxybutanedioic acid (0.066 g) was dissolved in acetone (3 ml) while heating. The two solutions were combined. More acetone (10 ml) was added and the mixture was boiled, then allowed to cool. The mixture was stirred at room temperature for 2 hours. The precipitate was filtered off (glass filter) and dried (vacuum; room temperature; 3 hours), yielding 126mg (74 %) of final compound 35.

### Example B12

### Preparation of final compound 3 and 4 (free base of final compound 3)

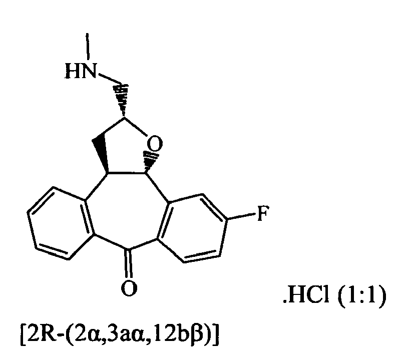

[2R-(2α,3aα,12bβ)]-2-N-(methylaminomethyl)-11-fluoro-3,3a,8,12b-tetrahydro-2*H-*dibenzo[3,4:6,7]cyclohepta[1,2-b]furan, described in WO03/048146, (0.00336 mol) was stirred in DCM (20 ml). A solution of potassium permanganate (0.01011 mol), potassium hydroxide (0.00169 mol) and *N,N,N,N*-tetrabuthylamonium sulfate (1:1) (0.00034 mol) in water (20 ml) was added dropwise. The reaction mixture was stirred for 22 hours at room temperature. The precipitate was filtered off over dicalite, rinsed with DCM and the layers were separated. The organic phase was washed once with a saturated aqueous NaHSO₃ solution. The organic layer was separated, and the water layer was extracted three times with DCM. The combined organic layers were washed with water, dried, filtered and the solvent evaporated (vacuum, 40°C). The residue (0.981 g) was dissolved in methanol, then purified by HPLC over Kromasil KR100-10 RP-18 (eluent: 0.2 % DIPA/(CH₃CN + 0.2 % DIPA) gradient elution). The product fractions were collected and the solvent was evaporated (Rotavap, 30 °C), yielding 0.269 g of final compound 4. This compound was stirred in diethyl ether (1 3 ml, p.a.) and converted into the hydrochloric acid salt (1:1) with 6 N HCl/2-propanol (0.150 ml). 2-Propanone (5 drops, p.a.) was added. The mixture was stirred for one hour at room temperature. The precipitate was filtered off and dried (vacuum, 60 °C, 3 hours), yielding 0.200 g (17 %) of final compound 3.

### Example B13

### Preparation of final compound 104

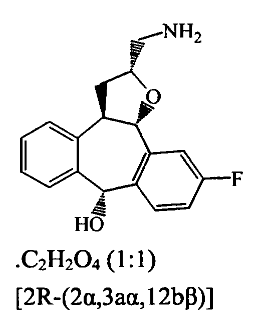

A solution of intermediate compound 25 (0.00046 mol) in MeOH (100 ml) was hydrogenated at room temperature for 30 minutes with Pd/C (0.5 g) as a catalyst. After uptake of H₂ (1 equiv.), the resulting suspension was filtered over celite and the filtrate was evaporated under reduced pressure. The residual oil was purified by short open column chromatography (eluent: DCM/(MeOH/NH₃) 9/1). The product fractions were collected and the solvent was evaporated. The obtained residue was converted into the ethanedioic acid salt and then the resulting solids were collected, yielding 0.026 g of final compound 104.

### Example B14

### Preparation of final compound 122

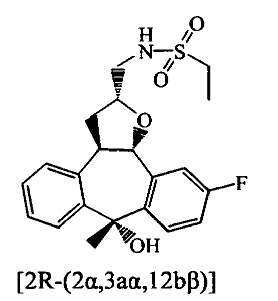

A mixture of final compound 112 (made according to B 13) (0.00049 mol), ethanesulfonyl chloride (0.00054 mol) and triethylamine (0.00064 mol) in DCM (10 ml) was stirred for 2 hours at room temperature and then the reaction mixture was partitioned between water (30 ml) and DCM (50 ml). The organic layer was washed with water (30 ml) and with brine (30 ml), dried (Na₂SO₄), filtered off and the solvent was evaporated. The residual oil was purified by short open column chromatography (eluent: DCM). The product fractions were collected and the solvent was evaporated, yielding 0.085 g of final compound 122.

### Example B15

a) Preparation of final compound 131 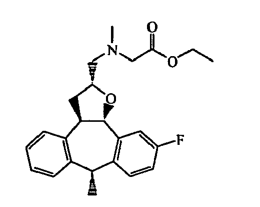 A mixture of final compound 75 (made according to final compound 4) (0.00086 mol), chloro- acetic acid, ethyl ester (0.00096 mol) and potassium carbonate (0.00257 mol) in acetonitrile (5 ml) was heated for 10 minutes at 150 °C under microwave irradiation. The mixture was cooled to room temperaturs and DCM was added. The mixture was filtered. The filter residue was washed with DCM. The filtrate's solvent was evaporated. The residue was purified by short open column chromatography in a Manifold (eluent: DCM/EtOAc 100/0 and 80/20). The product fractions were collected and the solvent was evaporated, yielding 0.307 g (90%) of final compound 131.
b) Preparation of final compound 103 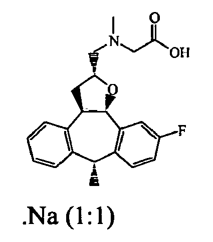 A solution of sodium hydroxide (0.00085 mol) in water (1 ml) was added to final compound 131 (0.00077 mol) in dioxane (10 ml). The reaction mixture was stirred for 24 hours at room temperature. Na₂SO₄ was added. The solid was removed by filtration. The filtrate's solvent was evaporated. The residue was treated with diethyl ether, filtered off and dried, yielding 0.255 g (85 %) of final compound 103.

Tables 1 and 2 list compounds of Formula (I) which were prepared according to one of the above examples.

**Table 1**

| | | | | | | | | |
|---|---|---|---|---|---|---|---|---|
| 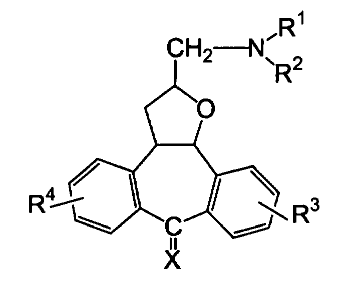 | | | | | | | | |

| **Co. No.** | **Ex. No.** | **x** | **R¹** | **R²** | **R³** | **R⁴** | **Stereochemical / salt data** | **Melting point (°C)** |
|---|---|---|---|---|---|---|---|---|
| **1** | 6 | =O | -H | -H | 11-F | -H | [2R-(2α,3aα,12bβ)] | |
| **11** | 6 | =O | -H | -H | 11-F | -H | .C₂H₂O₄(1:1); [2R-(2α,3aα,12bβ)] | |
| **2** | 6 | =O | -H | -OCH₃ | 11-F | -H | [2R-(2α,3aα,12bβ)] | |
| **4** | 11 | =O | -CH₃ | -H | 11-F | -H | [2R-(2α,3aα,12bβ)] | |
| **3** | 11 | =O | -CH₃ | -H | 11-F | -H | .HCl (1:1); [2R-(2α,3aα,12bβ)] | |
| **6** | 4 | =O | -CH₃ | -CH₃ | -H | -H | (2β,3aα,12bβ) | |
| **5** | 4 | =O | -CH₃ | -CH₃ | -H | -H | .C₂H₂O₄(1:1); (2β,3aα,12bβ) | |
| **7** | 4 | =O | -CH₃ | -CH₃ | -H | -H | [2R*-(2α,3aα,12bβ)] | |
| **8** | 4 | =O | -CH₃ | -CH₃ | -H | -H | [2R*-(2α,3aβ,12bα)] | |
| **9** | 4 | =O | -CH₃ | -CH₃ | -H | -H | [2S*-(2α,3aα,12bβ)] | |
| **10** | 4 | =O | -CH₃ | -CH₃ | -H | -H | [2S*-(2α,3aβ,12bα)] | |
| **87** | 4 | =O | -CH₃ | -CH₃ | -H | -H | (2α,3aα,12bβ) + (2β,3aα,12bβ) | |
| **13** | 6 | =O | -CH₃ | -CH₃ | 11-F | -H | [2R-(2α,3aα,12bβ)] | |
| **12** | 6 | =O | -CH₃ | -CH₃ | 11-F | -H | .C₂H₂O₄(1:1); [2R-(2α,3aα,12bβ)] | |
| **14** | 8 | =O | -CH₃ | -CH₃ | 11-Cl | -H | [2R-(2α,3aα,12bβ)] | |
| **86** | 6 | =O | -CH₃ | -CH₃ | 11-Br | -H | [2R-(2α,3aα,12bβ)] | |
| **15** | 6 | =O | -CH₃ | -CH₃ | 11-OCH₃ | -H | [2R-(2α,3aα,12bβ)]+ [2S-(2β,3aα,12bβ)] | |
| **16** | 6 | =O | -CH₃ | -CH₃ | 11-OCH₃ | -H | [2R-(2α,3aα,12bβ)] | |
| **18** | 7 | =O | -CH₃ | -CH₃ | 11-NH₂ | -H | [2R-(2α,3aα,12bβ)] | |
| **17** | 7 | =O | -CH₃ | -CH₃ | 11-NH₂ | -H | .C₂H₂O₄(1:1); [2R-(2α,3aα,12bβ)] | |
| **19** | 7 | =O | -CH₃ | -CH₃ | 11-NHCH₃ | -H | [2R-(2α,3aα,12b)] | |
| **20** | 5 | =O | -CH₃ | -CH₃ | 11-N(CH₃)₂ | -H | [2R-(2α,3aα,12bβ)] | |
| **90** | 13 | =CH₂ | -H | -H | 11-F | -H | [2R-(2α,3aα,12bβ)] | |
| **91** | 4 | =CH₂ | -H | -Ethyl | 11-F | -H | [2R-(2α,3aα,12bβ)] | |
| **92** | 4 | =CH₂ | -H | -Phenyl | 11-F | -H | [2R-(2α,3aα,12bβ)] | |
| **93** | 4 | =CH₂ | -CH₃ | -H | -H | 5-F | .C₂H₂O₄(1:1); [2R*-(2α,3aα,12bβ)] | 201 |
| **94** | 4 | =CH₂ | -CH₃ | -H | -H | 5-F | .C₂H₂O₄(1:1); [2R*-(2α,3αβ,12bα)] | 211.9 |
| **95** | 4 | =CH₂ | -CH₃ | -H | 11-F | -H | .C₂H₂O₄(1:1); [2S-(2α,3aα,12bβ)] | 192.8 |
| **96** | 2 | =CH₂ | -CH₃ | -H | 11-F | 6-F | .C₂H₂O₄(1:1) ; [2RS-(2α,3aα,12bβ)] | |
| **97** | 2 | =CH₂ | -CH₃ | -H | 11-F | 6-F | .C₂H₂O₄(1:1); [2RS-(2β,3aα,12bβ)] | 183.9 |
| **98** | 2 | =CH₂ | -CH₃ | -H | 10-F | 5-F | .C₂H₂O₄(1:1); [2RS-(2β,3aα,12bβ)] | |
| **21** | 4 | =CH₂ | -CH₃ | -CH₃ | -H | -H | [2R*-(2α,3aα,12bα)] | |
| **22** | 4 | =CH₂ | -CH₃ | -CH₃ | -H | -H | [2R*-(2α,3αβ,12bβ)] | |
| **23** | 4 | =CH₂ | -CH₃ | -CH₃ | -H | -H | [2S*-(2α,3aα,12bα)] | |
| **24** | 4 | =CH₂ | -CH₃ | -CH₃ | -H | -H | [2S*-(2α,3aβ,12bβ)] | |
| **25** | 2 | =CH₂ | -CH₃ | -CH₃ | -H | -H | [2R-(2α,3aα,12bβ)] | |
| **89** | 4 | =CH₂ | -CH₃ | -CH₃ | -H | -H | (2α,3aα,12bα) + (2β,3aα,12bα) | |
| **26** | 2 | =CH₂ | -CH₃ | -CH₃ | 11-F | -H | [2R-(2α,3aα,12bβ)] | |
| **27** | 2 | =CH₂ | -CH₃ | -CH₃ | 11-F | -H | .C₂H₂O₄(1:1); [2R-(2α,3aα,12bβ)] | |
| **28** | 2 | =CH₂ | -CH₃ | -CH₃ | 11-F | 5-F | (2β,3aα,12bβ) | |
| **29** | 2 | =CH₂ | -CH₃ | -CH₃ | 11-F | 5-F | .C₂H₂O₄(1:1); (2β,3aα,12bβ) | 210.2 |
| **30** | 2 | =CH₂ | -CH₃ | -CH₃ | 11-OCH₃ | -H | [2R-(2α,3aα,12bβ)] | |
| **88** | 2 | =CH₂ | -CH₃ | -CH₃ | 11-OCH₃ | -H | [2R-(2α,3aα,12bβ)] | |
| **32** | 2 | =CH₂ | -CH₃ | -CH₃ | 11-N(CH₃)₂ | -H | [2R-(2α,3aα,12bβ)] | |
| **31** | 2 | =CH₂ | -CH₃ | -CH₃ | 11-N(CH₃)₂ | -H | .C₂H₂O₄(1:1); [2R-(2α,3aα,12bβ)] | 169.2 |

**Table 2**

| | | | | | | | | | | |
|---|---|---|---|---|---|---|---|---|---|---|
| 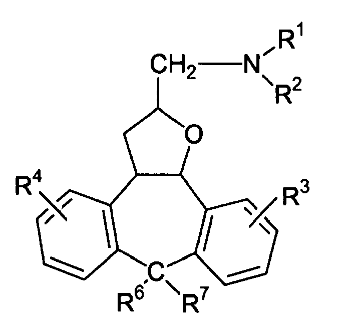 | | | | | | | | | | |

| **Co. No.** | **Ex. No.** | **R⁶** | **R⁷** | **R¹** | **R²** | **R³** | **R⁴** | **Stereochemical / salt data** | **Melting point (°C)** | **Optical rotation (αD)** |
|---|---|---|---|---|---|---|---|---|---|---|
| **75** | 4 | -H | -CH₃ | -H | -CH₃ | 11-F | -H | [2R-(2α,3aα,8α,12bβ)] | 96 | |
| **72** | 4 | -H | -CH₃ | -H | -CH₃ | 11-F | -H | [2R-(2α,3aα,8β,12bβ)] | | |
| **73** | 4 | -H | -CH₃ | -H | -CH₃ | 11-F | -H | [2R-(2α,3aα,8β,12bβ)] C₂H₂O₄(1:1) | 176 | |
| **74** | 4 | -H | -CH₃ | -H | -CH₃ | **11-F** | -H | [2R-(2α,3aα,8α,12bβ)] .HCl(1:1) | | +101.4° (Na), c 0.54 |
| **99** | 4 | -H | -CH₃ | -H | -CH₃ | 11-F | -H | [2S-(2α,3aα,8β,12bβ)] + [2S-(2α,3aα,8α,12bβ)] mixture 70/30 of epimers .C₄H₆O₆(1:1) | 187.1 | |
| **100** | 4 | -H | -CH₃ | -H | -CH₃ | 11-F | -H | [2R*-(2β,3aα,8β,12bβ)] .C₄H₆O₆ | 211.7 | |
| **101** | 4 | -H | -CH₃ | -H | -CH₃ | -H | 5-F | [2R-(2α,3aα,8α,12bβ)] .C₄H₆O₆ | 147.2 | |
| **102** | 4 | -H | -CH₃ | -H | -CH₃ | -H | 5-F | [2R-(2α,3aβ,8α,12bα)] + [2R-(2α,3aβ,8β,12bα)] mixture 60/40 of epimers .C₄H₆O₆ (1:1) | 192.1 | |
| **71** | 3 | -H | -CH₃ | -CH₃ | -CH₃ | -H | -H | [2R-(2α,3aα,8α,12bβ)] | | |
| **70** | 3 | -H | -CH₃ | -CH₃ | -CH₃ | -H | -H | [2R-(2α,3aα,8β,12bβ] | | |
| **68** | 3 | -H | -CH₃ | -CH₃ | -CH₃ | 11-F | -H | [2R-(2α,3aα,8α,12bβ)] | 90 | |
| **65** | 4 | -H | -CH₃ | -CH₃ | -CH₃ | 11-F | -H | [2R-(2α,3aα,8β,12bβ] | | |
| **69** | 3 | -H | -CH₃ | -CH₃ | -CH₃ | 11-F | -H | [2R-(2α,3aα,8α,12bβ) +[2R-(2α,3aα,8β,12bβ)] | | |
| **66** | 3 | -H | -CH₃ | -CH₃ | -CH₃ | 11-F | -H | [2R-(2α,3αα,8α,12bβ)] .C₂H₂O₄(1:1) | 192 | +67° (Na), c 0.22 |
| **64** | 3 | -H | -CH₃ | -CH₃ | -CH₃ | 11-F | -H | [2R-(2α,3aα,8β,12bβ)] .C₂H₂O₄ (1:1) | | |
| **67** | 4 | -H | -CH₃ | -CH₃ | -CH₃ | 11-F | -H | [2R-(2α,3aα,8α,12bβ)] .HCl(1:1) | | |
| **76** | 3 | -H | -CH₃ | -CH₃ | -CH₃ | 11-F | 5-F | (2β,3aα,8α,12bβ) +(2β,3aα,8β,12bβ)] | | |
| **77** | 3 | -H | -CH₃ | -CH₃ | -CH₃ | 11-F | 5-F | [2R*-(2β,3aα,8α,12bβ)] +[2R*-(2α,3aα,8β,12bβ)] .C₂H₂O₄(1:1) | 79 | |
| **78** | 3 | -H | -C^{H}₃ | -CH₃ | -CH₃ | 11-N(CH₃)₂ | -H | [2R-(2α,3aα,8α,12bβ)] | | |
| **79** | 3 | -H | -CH₃ | -CH₃ | -CH₃ | 11-N(CH₃)₂ | -H | [2R-(2α,3aα,8β,12bβ)] | | |
| **103** | 15 | -H | -CH₃ | -CH₃ | -CH₂CO₂Na | 11-F | -H | [2R-(2α,3aα,8α,12bβ)] | | |
| **131** | 15 | -H | -CH₃ | -CH₃ | -CH₂CO₂Et | 11-F | -H | [2R-(2α,3aα,8α,12bβ)] | | |
| **104** | 13 | -OH | -H | -H | -H | 11-F | -H | [2R-(2α,3aα,8α,12bβ)] | | |
| **105** | 13 | -OH | -H | -H | -H | 11-F | -H | [2R-(2α,3aα,8β,12bβ)] | | |
| **106** | 4 | -OH | -H | -H | -Ethyl | 11-F | -H | [2RS-(2α,3aα,8α,12bβ)] | | |
| **107** | 4 | -OH | -H | -H | -Ethyl | 11-F | -H | [2R-(2α,3aα,8β,12bβ)] | | |
| **108** | 4 | -OH | -H | -H | -CH₂-Ph | 11-F | -H | [2R-(2α,3aα,8α,12bβ)] | | |
| **109** | 4 | -OH | -H | -H | -CH₂-Ph | 11-F | -H | [2R-(2α,3aα,8β,12bβ)] | | |
| **60** | 4 | -OCH₃ | -H | -CH₃ | -CH₃ | 11-F | -H | [2R-(2α,3aα,8α,12bβ)]+ [2R-(2α,3aα,8β,12bβ)] .C₄H₆O₆ (1:1) | 188 | |
| **110** | 4 | -OCH₃ | -H | -H | -Ethyl | 11-F | -H | [2R-(2α,3αα,8α,12bβ)] | | |
| **111** | 4 | -OCH₃ | -H | -H | -CH₂-Ph | 11-F | -H | [2R-(2α,3aα,8α,12bβ)] | | |
| **63** | 10 | -OCH(CH₃)₂ | -H | -H | -CH₃ | 11-F | -H | [2R-(2α,3aα,8α,12bβ)] .HCl(1:1) | | |
| **112** | 13 | -OH | -CH₃ | -H | -H | 11-F | -H | [2R-(2α,3aα,8α,12bβ)] | | |
| **113** | 13 | -OH | -CH₃ | -H | -H | 11-F | -H | [2R-(2α,3aα,8β,12bβ)] | | |
| **114** | 4 | -OH | -CH₃ | -H | -CH₃ | -H | 5-F | [2R*-(2α,3aβ,8β,12bα)] | 212.3 | |
| **115** | 4 | -OH | -CH₃ | -H | -CH₃ | -H | 5-F | [2R*-(2α,3aβ,8α,12bα)] | | |
| **116** | 4 | -OH | -CH₃ | -H | -CH₃ | 10-F | 5-H | [2RS-(2β,3aα,8α,12bβ)] + [2RS-(2β,3aα,8β,12bβ)] 1:1 mixture .C₄H₆O₆ (1:1) | 175.2 | |
| **117** | 4 | -OH | -CH₃ | -H | -CH₃ | 10-F | 5-H | [2RS-(2α,3aα,8α,12bβ)] + [2RS-(2α,3aα,8β,12bβ)] .C₄H₆O₆ (1:1) | 171 | |
| **118** | 4 | -OH | -CH₃ | -H | -Et | 11-F | -H | [2R-(2α,3aα,8α,12bβ)] | | |
| **119** | 4 | -OH | -CH₃ | -H | -Et | 11-F | -H | [2R-(2α,3aα,8β,12bβ)] | | |
| **120** | 4 | -OH | -CH₃ | -H | -Ph | 11-F | -H | [2R-(2α,3aα,8α,12bβ)] | | |
| **121** | 4 | -OH | -CH₃ | -H | -Ph | 11-F | -H | [2R-(2α,3aα,8β,12bβ)] | | |
| **122** | 14 | -OH | -CH₃ | -H | 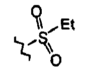 | 11-F | -H | [2R-(2α,3aα,8α,12bβ)] | | |
| **123** | 14 | -OH | -CH₃ | -H | 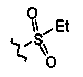 | 11-F | -H | [2R-(2α,3aα,8β,12bβ)] | | |
| **124** | 4 | -OH | -CH₃ | -H | -CH₃ | 11-F | -H | [2S-(2α,3aα,8α,12bβ)] | 205.7 | |
| **125** | 4 | -OH | -CH₃ | -H | -CH₃ | 11-F | -H | [2S-(2α,3aα,8β,12bβ)] | 208.8 | |
| **126** | 4 | -OH | -CH₃ | -H | -CH₃ | 11-F | -H | [2R*-(2β,3aα,8α,12bβ)] | 191.6 | |
| **127** | 4 | -OH | -CH₃ | -H | -CH₃ | 11-F | 6-F | [2RS-(2α,3aα,8α,12bβ)] + [2RS-(2α,3aα,8β,12bβ)] 54:46 mixture .C₄H₆O₆(1:1) | 215.4 | |
| **128** | 4 | -OH | -CH₃ | -H | -CH₃ | 11-F | 6-F | [2RS-(2β,3aα,8α,12bβ)] + [2R-(2β,3aα,8β,12bβ)] .C₄H₆O₆(1:1) | 193.7 | |
| **129** | 4 | -OH | -CH₃ | -H | -CH₂-Ph | 11-F | -H | [2R-(2α,3aα,8α,12bβ)] | | |
| **130** | 4 | -OH | -CH₃ | -H | -CH₂-Ph | 11-F | -H | [2R-(2α,3aα,8β,12bβ)] | | |
| **62** | 4 | -OCH₃ | -CH₃ | -H | -CH₃ | 11-F | -H | [2R-(2α,3aα,8α,12bβ)] | | |
| **61** | 4 | -OCH₃ | -CH₃ | -H | -CH₃ | 11-F | -H | [2R-(2α,3aα,8β,12bβ)] | | |

### Analytical data

The compounds in Table 3 below were analysed by high pressure liquid chromatography/ high resolution mass spectroscopy (LCMS) in accordance with the following procedure.

The HPLC gradient was supplied by a HP 1100 from Agilent with a column heater set at 40°C. Flow from the column was passed through photodiode array (PDA) detector and then split to a Light Scattering detector (ELSD and to a Waters-Micromass Time of Flight (ToF) mass spectrometer with an electrospray ionization source operated simultaneously in positive and negative ionization mode.

Reversed phase HPLC was carried out on a XDB-C18 cartridge (3.5 µm, 4.6 x 30 mm) from Agilent, with a flow rate of 1 ml/min. Three mobile phases (mobile phase A: 0.5 g/l ammoniumacetate solution, mobile phase B: acetonitrile; mobile phase C: methanol) were employed to run a gradient condition from 80 % A, 10% B,10% C to 50% B and 50% C in 6.0 min., to 100 % B at 6.5 min., kept till 7.0 min and reequilibrated with 80 % A, 10% B and 10%C at 7.6 min. that was kept till 9.0 min. An injection volume of 5 µL was used.

High Resolution Mass spectra were acquired by scanning from 100 to 750 in 1s using a dwell time of 1 s. The capillary needle voltage was 3kV and the source temperature was maintained at 140°C. Nitrogen was used as the nebulizer gas. Cone voltage was 30 V for both positive and negative ionzation mode. Leucine-enkephaline was the reference used for the lock spray. Data acquisition was performed with a Waters-Micromass MassLynx-Openlynx data system.

The results of the analyses are given in Table 3 in which the mass peak detected corresponds in each case to the free base +H⁺.

**Table 3**

| **Co. No.** | **Retention time** | **Peak mass detected** | **Remarks** |
|---|---|---|---|
| **5** | 3.53 | 308 | |
| **7** | 3.84 | 308 | |
| **8** | 3.73 | 308 | |
| **9** | 3.85 | 308 | |
| **10** | 3.7 | 308 | |
| **15** | 5.78/ 5.99 | 338/338 | diastereoisomeric mixture |
| **17** | 2.76 | 323 | |
| **21** | 4.12 | 306 | |
| **22** | 4.29 | 306 | |
| **23** | 4.1 | 305 | |
| **24** | 4.26 | 306 | |
| **25** | 4.73 | 306 | |
| **26** | 4.94 | 324 | |
| **27** | 4.79 | 324 | |
| **28** | 4.83 | 342 | |
| **31** | 4.99 | 349 | |
| **40** | 3.48 | 342 | |
| **41** | 3.05 | 342 | |
| **44** | 2.96 | 354 | |
| **45** | 2.16 | 328 | |
| **47** | 3.32 | 360 | |
| **48** | 2.89 | 324 | |
| **49** | 2.77 | 324 | |
| **50** | 2.82 | 324 | |
| **51** | 2.83 | 324 | |
| **52** | 2.85 | 324 | |
| **53** | 2.88 | 324 | |
| **54** | 2.84 | 324 | |
| **55** | 2.85 | 324 | |
| **56** | 3.67 | 356 | |
| **57** | 3.94 | 356 | |
| **58** | 4.22 | 370 | |
| **59** | 4.07 | 342 | |
| **60** | 4.1 | 342 | |
| **61** | 4.02 | 342 | |
| **62** | 4.13 | 342 | |
| **64** | 4.69 | 325 | |
| **66** | 4.57 | 326 | |
| **70** | 4.55 | 307 | |
| **71** | 4.76 | 307 | |
| **72** | 3.92 | 312 | |
| **74** | 3.87 | 312 | |
| **76** | 4.62/4.69 | 344/344 | diastereoisomeric mixture |
| **78** | 4.88 | 351 | |
| **79** | 5.12 | 351 | |
| **80** | 2.97 | 354 | |
| **90** | 4.1 | 296 | |
| **91** | 4.3 | 324 | |
| **92** | 6.55 | 372 | |
| **93** | 4.19 | 310 | |
| **94** | 4.09 | 310 | |
| **95** | 4.16 | 310 | |
| **96** | 4.29 | 328 | |
| **97** | 4.21 | 328 | |
| **98** | 4.18 | 328 | |
| **99** | 4.05 | 312 | |
| **100** | 4.06 | 312 | |
| **101** | 4.00 | 312 | |
| **102** | 3.91/3.99 | 312 | diastereoisomeric mixture |
| **103** | 361 | 370 | |
| **104** | 2.67 | 300 | |
| **105** | 2.44 | 300 | |
| **106** | 2.82 | 328 | |
| **107** | 2.79 | 328 | |
| **108** | 4.86/4.93 | 390 | diastereoisomeric mixture |
| **109** | 4.87 | 390 | |
| **110** | 3.83 | 342 | |
| **111** | 5.62/5.67 | 404 | diastereoisomeric mixture |
| **112** | 2.35 | 314 | |
| **113** | 2.46 | 314 | |
| **114** | 2.49 | 328 | |
| **115** | 2.54 | 328 | |
| **116** | 2.75/2.83 | 346 | diastereoisomeric mixture |
| **117** | 2.81 | 346 | |
| **118** | 2.87 | 342 | |
| **119** | 2.92 | 342 | |
| **120** | 5.63 | 391 | |
| **121** | 5.64 | 390 | |
| **122** | 4.14 | 406 | |
| **123** | 4.14 | 406 | |
| **124** | 2.45 | 328 | |
| **125** | 2.53 | 328 | |
| **126** | 2.35/2.62 | 328 | diastereoisomeric mixture |
| **127** | 2.79/2.86 | 346 | diastereoisomeric mixture |
| **128** | 2.73/2.83 | 346 | diastereoisomeric mixture |
| **129** | 5.03 | 404 | |
| **130** | 4.96 | 404 | |

### C. Pharmacological data

### Example C1: in vitro binding affinity for 5-HT_{2A} and 5-HT_{2C} receptors

The interaction of the compounds of Formula (I) with 5-HT_{2A} and 5-HT_{2C} receptors was assessed in *in vitro* radioligand binding experiments. In general, a low concentration of a radioligand with a high binding affinity for the receptor is incubated with a sample of a tissue preparation enriched in a particular receptor (1 to 5 mg tissue) in a buffered medium (0.2 to 5 ml). During the incubation, the radioligands bind to the receptor. When equilibrium of binding is reached, the receptor bound radioactivity is separated from the non-bound radioactivity, and the receptor bound activity is counted. The interaction of the test compounds with the receptors is assessed in competition binding experiments. Various concentrations of the test compound are added to the incubation mixture containing the tissue preparation and the radioligand. Binding of the radioligand will be inhibited by the test compound in proportion to its binding affinity and its concentration. The affinities of the compounds for the 5-HT₂ receptors were measured by means of radioligand binding studies conducted with: (a) human cloned 5-HT_{2A} receptor, expressed in L929 cells using [¹²⁵I]R91150 as radioligand and (b) human cloned 5-HT_{2C} receptor, expressed in CHO cells using [³H]mesulergine as radioligand.

### Example C2: in vitro assay for NET inhibition

Cortex from rat brain was collected and homogenised using an Ultra-Turrax T25 and a Dual homogeniser in ice-cold homogenising buffer containing Tris, NaCl and KCl (50 mM, 120 mM and 5 mM, respectively, pH 7.4) prior to dilution to an appropriate protein concentration optimised for specific and non-specific binding. Binding was performed with radioligand [³H]Nixosetine (NEN, NET-1084, specific activity ~70 Ci/mmol) diluted in ice cold assay buffer containing Tris, NaCl and KCl (50 mM, 300 mM and 5 mM, respectively, pH 7.4). at a concentration of 20 nmol/L. Prepared radioligand (50 µl) was then incubated (60 min, 25 °C) with membrane preparations pre-diluted to an appropriate protein concentration (400 µl), and with 50 µl of either the 10 % DMSO control, Mazindol (10⁻⁶ mol/L final concentration), or test compound. Membrane-bound activity was detected by filtration through a Packard Filtermate harvester onto GF/B Unifilterplates, washed with ice-cold Tris-HCl buffer, containing NaCl and KCl (50 mM, 120mM and 4mM; pH7.4; 6 x 0.5 ml). Filters were allowed to dry for 24 h before adding scintillation fluid. Scintillation fluid was allowed to saturate filters for 24 h before counting in a Topcount scintillation counter. Percentage specific bound and competition binding curves were calculated using S-Plus software (Insightful).

### Example C3: in vitro assay for D₂ receptor binding

Frozen membranes of human dopamine D_{2L} receptor-transfected CHO cells were thawed, briefly homogenised using an Ultra-Turrax T25 homogeniser and diluted in Tris-HCl assay buffer containing NaCI, CaCl₂, MgCl₂, KCl (50, 120, 2, 1, and 5 mM respectively, adjusted to pH 7.7 with HCl) to an appropriate protein concentration optimised for specific and non-specific binding. Radioligand [³H]Spiperone (NEN, specific activity ~70 Ci/mmol) was diluted in assay buffer at a concentration of 2 nmol/L. Prepared radioligand (50 µl), along with 50 µl of either the 10 % DMSO control, Butaclamol (10⁻⁶mol/l final concentration), or compound of interest, was then incubated (30 min, 37 °C) with 400 µl of the prepared membrane solution. Membrane-bound activity was filtered through a Packard Filtermate harvester onto GF/B Unifilterplates and washed with ice-cold Tris-HCl buffer (50 mM; pH 7.7; 6 x 0.5 ml). Filters were allowed to dry before adding scintillation fluid and counting in a Topcount scintillation counter. Percentage specific bound and competition binding curves were calculated using S-Plus software (Insightful).

**Table 4**

| | | | | | |
|---|---|---|---|---|---|
| The results from the above assays are given in the following table as (pIC₅₀) values: "n.d." means "not determined". This Table demonstrates also the lower ratio of D₂/NET inhibition for compounds in accordance with the invention in comparison with Compound 2 in WO 03/048146 referred to above, designated as Compound A in the Table. | | | | | |

| **Co. No.** | **5-HT_{2A}** | **5-HT_{2C}** | **D₂** | **NET-inhibition** | **D₂/NET** |
|---|---|---|---|---|---|
| **A** | 8.9 | 9.2 | 7.5 | 7.8 | 0.96 |
| **73** | 8.7 | 8.7 | 7.2 | 7.4 | 0.96 |
| **43** | n.d. | 7.2 | 6.0 | 6.3 | 0.96 |
| **111** | n.d. | 7.1 | 5.5 | 5.7 | 0.96 |
| **58** | 7.6 | 7.9 | 6.5 | 6.8 | 0.95 |
| **16** | n.d. | 7.3 | 6.0 | 6.3 | 0.95 |
| **70** | 7.9 | 8.5 | 6.9 | 7.3 | 0.94 |
| **105** | 8.0 | 8.4 | 5.2 | 5.6 | 0.92 |
| **63** | 6.5 | 7.0 | < 5 | 5.4 | < 0.92 |
| **109** | n.d. | 7.9 | < 5 | 5.4 | < 0.92 |
| **118** | n.d. | 7.0 | 5.7 | 6.2 | 0.91 |
| **59** | n.d. | 7.3 | < 6 | 6.6 | < 0.91 |
| **78** | n.d. | 7.0 | 5.7 | 6.4 | 0.88 |
| **125** | n.d. | 5.2 | <5 | 5.7 | < 0.88 |
| **45** | n.d. | 8.1 | 6.4 | 7.3 | 0.87 |
| **41** | 7.5 | 7.3 | <6 | 6.9 | <0.87 |
| **66** | 7.6 | 8.4 | 6.5 | 7.5 | 0.86 |
| **107** | 7.7 | 8.2 | 5.8 | 6.8 | 0.86 |
| **25** | 7.9 | 8.4 | 6.7 | 7.9 | 0.85 |
| **115** | n.d. | 5.8 | <5 | 5.9 | < 0.85 |
| **56** | n.d. | 7.3 | 5.1 | 6.0 | 0.84 |
| **12** | 7.2 | 7.1 | <6 | 7.2 | <0.83 |
| **114** | n.d. | 6.0 | <5 | 6.2 | <0.83 |
| **44** | n.d. | 6.9 | <5 | 6.2 | <0.81 |
| **77** | n.d. | 7.3 | <6 | 7.5 | <0.80 |
| **60** | n.d. | 7.4 | 5.4 | 6.9 | 0.79 |
| **124** | n.d. | 5.2 | <5 | 6.5 | <0.77 |
| **34** | 7.5 | 8.3 | <6 | 7.7 | <0.77 |
| **113** | n.d. | 8.1 | <5 | 6.4 | <0.77 |
| **74** | 7.9 | 8.4 | 6.2 | 8.3 | 0.74 |
| **93** | n.d. | 8.4 | 6 | 8.2 | 0.73 |
| **71** | 6.9 | 7.8 | 5.9 | 8.0 | 0.73 |
| **23** | 7.6 | 7.1 | 5.7 | 7.9 | 0.72 |
| **29** | n.d. | 7.9 | <6 | 8.3 | <0.72 |
| **51** | 5.9 | 7.2 | <5 | 7.1 | <0.70 |
| **101** | n.d. | 7.7 | 5.4 | 7.8 | 0.69 |
| **126** | n.d. | 6.3 | < 5 | 7.2 | <0.69 |
| **99** | n.d. | 6.7 | <5 | 7.2 | <0.69 |
| **102** | n.d. | 7.3 | 5.1 | 7.5 | 0.68 |
| **106** | 7.7 | 8.3 | 5.9 | 8.4 | 0.67 |
| **95** | n.d. | 7.3 | 5.2 | 7.8 | 0.67 |
| **119** | n.d. | 7.7 | 5.0 | 7.5 | 0.67 |
| **94** | n.d. | 7.7 | 5.3 | 8.2 | 0.65 |
| **24** | 6.8 | 7.6 | <5 | 7.7 | <0.65 |
| **100** | n.d. | 6.8 | <5 | 8.1 | <0.62 |
| **110** | 5.9 | 6.4 | <5 | 8.0 | <0.62 |
| **104** | 7.9 | 8.6 | 5.1 | 6.7 | 0.60 |

### Example C4: Metabolic Stability Assay

Compounds according to the invention were tested for metabolic stability in the following assay using human liver microsomes in comparison with prior art compound A, referred to above.

Sub-cellular tissue preparations are made by centrifugal separation after mechanical homogenization of tissue. Tissue is rinsed in ice cold 0.1 M Tris-HCl (pH 7.4) buffer to wash excess blood. Tissue is then blotted dry, weighed and chopped coarsely using surgical scissors. The tissue pieces are homogenized in 3 volumes of ice cold 0.1 M phosphate buffer (pH 7.4) for 7 x 10 sec. The vessel is kept in/on ice during the homogenization process. Tissue homogenates are centrifuged at 9000 x g for 20 minutes at 4 °C. The resulting supernatant can be stored at -80 °C and is designated 'S9'.

The S9-fraction may be centrifuged at 100.000 x g for 60 minutes (4°C) . The resulting supernatant is aspirated, aliquoted and designated 'cytosol'. The pellet is resuspended in 0.1 M phosphate buffer (pH 7.4) in a final volume of 1 mL per 0.5 g original tissue weight and designated 'microsomes'.
The incubation is carried out in the following system :

| | | control |
|---|---|---|
| Phosphate buffer (pH 7.4) | 0.1 M | 0.1 M |
| Test compound substrate | 5.0 µM | 5.0 µM |
| protein- active | 1.0 mg/ml | ------ |
| protein-inactivated* | -------- | 1.0 mg/mL |
| NADPH-generating system** | | |

| | | |
|---|---|---|
| *protein-inactived : "S9" or microsomes are heat inactivated (10 min at 95°C) ** NADPH-generating system comprises: 0.8 mM glucose-6-phosphate, 0.8 magnesium chloride and 0.8 U of glucose-6-phosphate dehydrogenase. | | |

The reaction is started by the addition of 0.8 mM NADP and incubated for 15 min. Total assay volume is 250 microliters.
The reaction is stopped by the addition of 2 volumes of DMSO (or acetonitrile).
The samples are centrifuged (10 min, 900 x g) and supernatant stored at room temperature (when stopped with DMSO; no longer than 24 h) or -20°C (when stopped with acetonitrile; no longer than 24 h) before analysis.
The supernatant material is then analysed by LC-MS to determine the extent of metabolism of the test compound in the microsomes
The results are given in the following Table 6.

**Table 6**

| **Compound** | **Stereochemistry** | **Salt form** | **% metabolised** |
|---|---|---|---|
| **Prior art compound A** | [2R-(2α,3aα,12bβ)] | Tartrate salt | 21.85 |
| **41** | [2R-(2α,3aα,8α,12bβ)] | Free base | 2.25 |
| **26** | [2R-(2α,3aα,12bβ)] | Free base | 2.6 |
| **66** | [2R-(2α,3aα,8α,12bβ)] | Oxalate salt | 11.35 |
| **70** | [2R-(2α,3aα,8β,12bβ] | Free Base | 13.5 |
| **40** | [2R-(2α,3aα,8α,12bβ)]- | Free Base | 4.0 |
| **71** | [2R-(2α,3aα,8α,12bβ)] | Free Base | 8 |
| **72** | [2R-(2α,3aα,8β,12bβ)] | Free Base | 6 |
| **74** | [2R-(2α,3aα,8α,12bβ)] | Hydrochloride salt | 9 |
| **80** | [2R-(2α,3aα,8aα,12b β)] | Free base | 1 |

### D. Composition examples

"Active ingredient" (A.I.) as used throughout these examples relates to a compound of Formula (I), a pharmaceutically acceptable acid addition salt, a stereochemically isomeric form thereof or a *N*-oxide form thereof.

### Example D.1 : ORAL SOLUTION

Methyl 4-hydroxybenzoate (9 g) and propyl 4-hydroxybenzoate (1 g) were dissolved in boiling purified water (41). In 3 1 of this solution were dissolved first 2,3-dihydroxybutanedioic acid (10 g) and thereafter A.I (20 g). The latter solution was combined with the remaining part of the former solution and 1,2,3-propanetriol (121) and sorbitol 70 % solution (3 1) were added thereto. Sodium saccharin (40 g) were dissolved in water (500 ml) and raspberry (2 ml) and gooseberry essence (2 ml) were added. The latter solution was combined with the former, water was added q.s. to a volume of 20 providing an oral solution comprising 5 mg of the active ingredient per teaspoonful (5 ml). The resulting solution was filled in suitable containers.

### Example D.2 : FILM-COATED TABLETS

### Preparation of tablet core

A mixture of A.I. (100 g), lactose (570 g) and starch (200 g) was mixed well and thereafter humidified with a solution of sodium dodecyl sulfate (5 g) and polyvinylpyrrolidone (10g) in water (200 ml). The wet powder mixture was sieved, dried and sieved again. Then there was added microcrystalline cellulose (100 g) and hydrogenated vegetable oil (15 g). The whole was mixed well and compressed into tablets, giving 10.000 tablets, each containing 10 mg of the active ingredient.

### Coating

To a solution of methyl cellulose (10 g) in denaturated ethanol (75 ml) there was added a solution of ethyl cellulose (5 g) in dichloromethane (150 ml). Then there were added dichloromethane (75 ml) and 1,2,3-propanetriol (2.5 ml). Polyethylene glycol (10 g) was molten and dissolved in dichloromethane (75 ml). The latter solution was added to the former and then there were added magnesium octadecanoate (2.5 g), polyvinylpyrrolidone (5 g) and concentrated colour suspension (30 ml) and the whole was homogenated. The tablet cores were coated with the thus obtained mixture in a coating apparatus.

### Example D.3 : INJECTABLE SOLUTION

Methyl 4-hydroxybenzoate (1.8 g) and propyl 4-hydroxybenzoate (0.2 g) were dissolved in boiling water (500 ml) for injection. After cooling to about 50°C there were added while stirring lactic acid (4 g), propylene glycol (0.05 g) and A.I. (4 g). The solution was cooled to room temperature and supplemented with water for injection q.s. ad 1000 ml, giving a solution comprising 4 mg/ml of A.I.. The solution was sterilized by filtration and filled in sterile containers.

## Claims

1. A compound of Formula (1) 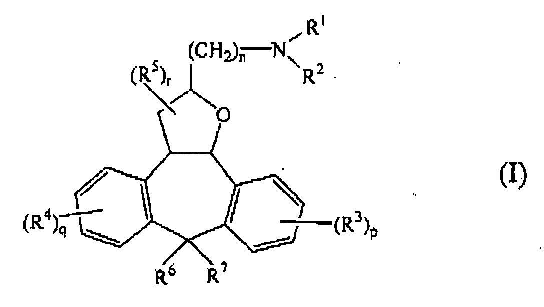 a pharmaceutically acceptable acid or base addition salt thereof, a stereochemically isomeric form thereof and an *N*-oxide form thereof, wherein :
n is an integer equal to zero, 1, 2, 3, 4, 5, or 6;
p is an integer equal to zero, 1, 2, 3 or 4;
q is an integer equal to zero, 1, 2, 3 or 4;
r is an integer equal to zero, 1, 2, 3, 4 or 5;
R¹ and R² each independently is selected from the group of hydrogen; C₁₋₆alkyl; C₁₋₆alkylcarbonyl; halomethylcarbonyl; aryl ; alkylsulphonyl and C₁₋₆alkyl substituted with hydroxy, C₁₋₆alkyloxy, carboxyl, C₁₋₆alkylcarbonyloxy, C₁₋₆alkyloxycarbonyl or aryl ;
or R¹ and R² taken together with the nitrogen atom to which they are attached may form a morpholinyl ring or a radical of Formula (a) to (e): 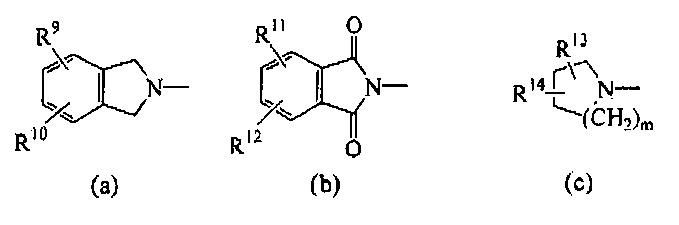 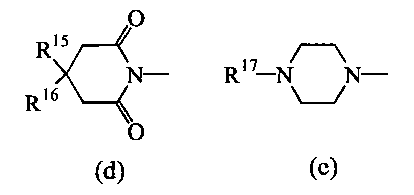 wherein :
R9, R¹⁰, R¹¹ and R¹² each independently are selected from the group of hydrogen ; halo ; halomethyl and C₁₋₆alkyl ;
m is an integer equal to zero, 1, 2, or 3;
R¹³, R¹⁴, R¹⁵ and R¹⁶ each independently are selected from the group of hydrogen ; C₁₋₆alkyl ; aryl and arylcarbonyl ; or R¹⁵ and R¹⁶ taken together may form a bivalent radical C₄₋₅alkanediyl ;
R¹⁷ is selected from the group of hydrogen; C₁₋₆alkyl; C₁₋₆alkylcarbonyl; halomethylcarbonyl; C₁₋₆alkyloxycarbonyl; aryl; di(aryl)methyl; C₁₋₆alkyl substituted with hydroxy, C₁₋₆alkyloxy, carboxyl, C₁₋₆alkylcarbonyloxy, C₁₋₆alkyloxycarbonyl and aryl;
each R³ independently is selected from the group of hydrogen ; halo ; cyano ; hydroxy ;halomethyl ; halomethoxy ; carboxyl ; nitro ; amino ; mono- or di(C₁₋₆alkyl)amino ; C₁₋₆alkylcarbonylamino ; aminosulfonyl ; mono- or di(C₁₋₆alkyl)aminosulfonyl ; C₁₋₆alkyl ; C₁₋₆alkyloxy ; C₁₋₆alkylcarbonyl and C₁₋₆alkyloxycarbonyl ;
each R⁴ independently is selected from the group of hydrogen ; halo ; cyano ; hydroxy ; halomethyl ; halomethoxy ; carboxyl ; nitro ; amino ; mono- or di(C₁₋₆alkyl)amino ; C₁₋₆alkylcarbonylamino ; aminosulfonyl ; mono- or di(C₁₋₆alkyl)aminosulfonyl ; C₁₋₆alkyl ; C₁₋₆alkyloxy ; C₁₋₆alkylcarbonyl and C₁₋₆alkyloxycarbonyl;
each R⁵ independently is selected from the group of C₁₋₆alkyl ; cyano and halomethyl;
R⁶ and R⁷ each independently from each other, are selected from the group of hydrogen, hydroxy, C₁₋₆alkyl, halomethyl and C₁₋₆alkyloxy ; with the proviso that R⁶ and R⁷ are not simultaneously hydrogen ; or R⁶ and R⁷ taken together may form a methylene radical (=CH₂); or, together with the carbon atom to which they are attached, a carbonyl; and
aryl is phenyl; or phenyl substituted with 1, 2 or 3 substituents selected from the group of halo, hydroxy, C₁₋₆alkyl and halomethyl.

2. A compound according to claim 1, **characterized in that** n is 1.

3. A compound according to any one of claims 1 to 2, **characterized in that**
n is an integer equal to 1;
p is an integer equal to zero or 1;
q is an integer equal to zero or 1;
r is an integer equal to zero;
R¹ and R² each independently is selected from the group of hydrogen; C₁₋₆alkyl ; aryl ; alkylsulphonyl and C₁₋₆alkyl substituted with carboxyl or aryl ;
R³ is selected from the group of hydrogen ; halo; amino ; mono- or di(C₁₋₆alkyl)amino and C₁₋₆alkyloxy;
R⁴ is hydrogen or halo ;
R⁶ and R⁷ each independently from each other, are selected from the group of hydrogen ; hydroxy ; C₁₋₆alkyl and C₁₋₆alkyloxy ; with the proviso that R⁶ and R⁷ are not simultaneously hydrogen ; or R⁶ and R⁷ taken together form methylene (=CH₂); or, together with the carbon atom to which they are attached, a carbonyl.

4. A compound according to any one of claims 1 to 3, **characterized in that** R¹ and R² are each independently selected from the group of hydrogen ; methyl ; ethyl; methoxy ; phenyl and benzyl.

5. A compound according to any one of claims 1 to 4, **characterized in that** both R¹ and R² are methyl ; or R¹ is hydrogen and R² is methyl.

6. A compound according to any one of claims 1 to 5, **characterized in that** p is zero or 1 and R³ is selected from the group of hydrogen ; fluoro ; chloro ; bromo ; methoxy ; amino ; methylamino and dimethylamino.

7. A compound according to any one of claims 1 to 6, **characterized in that** q is zero or 1 and R⁴ is selected from the group of hydrogen and fluoro.

8. A compound according to any one of claims 1 to 7, **characterized in that** R⁶ and R⁷ are selected from the group of hydrogen, methyl, ethyl, isopropyl, hydroxy, methoxy and isopropoxy; or R⁶ and R⁷ taken may form methylene; or, together with the carbon atom to which they are attached, a carbonyl.

9. A compound according to any one of claims 1 to 8, **characterized in that** the hydrogen atoms on carbon atoms 3a and 12b have a trans configuration or a compound according to any one of claims 1 to 8, **characterized in that** the compound has the (2α, 3aα, 12bβ) stereochemical configuration.

10. A compound according to any one of claims 1 to 9, **characterized in that** the compounds are selected from the group of compounds :
ο (5,11-difluoro-8-methylene-3,3a,8,12b-tetrahydro-2*H*-1-oxa-dibenzo-[e,h]azulen-2-ylmethyl)-dimethyl-amine ;
ο 11-fluoro-2-methylaminomethyl-3,3a,8,12b-tetrahydro-2*H*-1-oxa-dibenzo-[e,h]azulen-8-ol ;
ο 11-fluoro-8-methyl-2-methylaminomethyl-3,3a,8,12b-tetrahydro-2*H*-1-oxa-dibenzo[e,h]azulen-8-ol ;
ο 2-dimethylaminomethyl-8-methyl-3,3a,8,12b-tetrahydro-2*H*-1-oxa-dibenzo[e,h]azulen-8-ol;
ο (11-fluoro-8-methoxy-8-methyl-3,3a,8,12b-tetrahydro-2*H*-1-oxa-dibenzo[e,h]azulen-2-ylmethyl)-dimethyl-amine;
ο (11-fluoro-8-methyl-3,3a,8,12b-tetrahydro-2*H*-1-oxa-dibenzo[e,h]azulen-2-ylmethyl)-dimethyl-anline;
ο (8-methyl-3,3a,8,12b-tetrahydro-2*H*-1-oxa-dibenzo[e,h]azulen-2-ylmethyl)-dimethyl-amine; and
ο (8-methyl-3,3a,8,12b-tetrahydro-2*H*-1-oxa-dibenzo[e,h]azulen-2-ylmethyl)-methyl-amine.

11. A compound according to any one of claims 1-0 for use as a medicine.

12. The use of a compound according to any one of claims 1-10 for the manufacture of a medicament for treating serotonin-, dopamine- and norepinephrine-mediated conditions.

13. The use of a compound according to claim 12 for the manufacture of a medicament for the treatment and/or prophylaxis of anxiety, psychosis, depression, migraine and addictive properties of drugs of abuse.

14. A pharmaceutical composition comprising a pharmaceutically acceptable carrier and, as active ingredient, a therapeutically effective amount of a compound according to any one of claims 1-10.

15. A process for the preparation of a composition as claimed in claim 14, **characterized in that** a pharmaceutically acceptable carrier is intimately mixed with a therapeutically effective amount of a compound as claimed in any one of claims 1-10.

## Patentansprüche

1. Verbindungen der Formel (I) 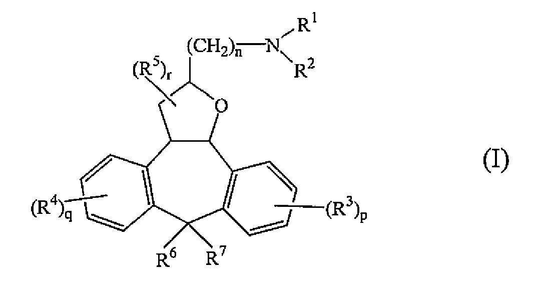 deren pharmazeutisch unbedenkliche Säure- oder Basenadditionssalze, deren stereochemisch isomere Formen und deren N-Oxidformen, wobei:
n für eine ganze Zahl gleich null, 1, 2, 3, 4, 5 oder 6 steht;
p für eine ganze Zahl gleich null, 1, 2, 3 oder 4 steht;
q für eine ganze Zahl gleich null, 1, 2, 3 oder 4 steht;
r für eine ganze Zahl gleich null, 1, 2, 3, 4 oder 5 steht;
R¹ und R² jeweils unabhängig voneinander ausgewählt sind aus der Gruppe bestehend aus Wasserstoff; C₁₋₆-Alkyl; C₁₋₆-Alkylcarbonyl; Halogenmethylcarbonyl; Aryl; Alkylsulfonyl und C₁₋₆-Alkyl substituiert durch Hydroxy, C₁₋₆-Alkyloxy, Carboxyl, C₁₋₆-Alkylcarbonyloxy, C₁₋₆-Alkyloxycarbonyl oder Aryl;
oder R¹ und R² zusammen mit dem Stickstoffatom, an das sie gebunden sind, einen Morpholinylring oder einen Rest der Formel (a) bis (e) bilden können: 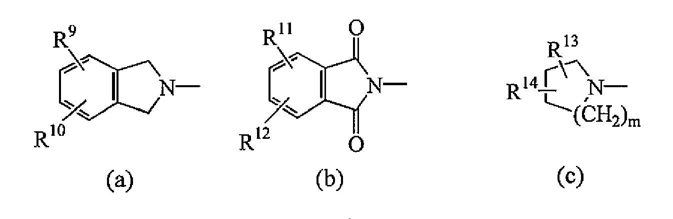 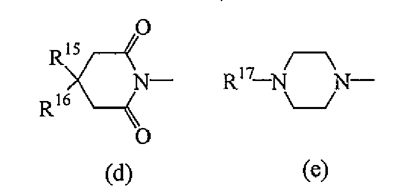 wobei:
R⁹, R¹⁰, R¹¹ und R¹² jeweils unabhängig ausgewählt sind aus der Gruppe bestehend aus Wasserstoff; Halogen; Halogenmethyl und C₁₋₆-Alkyl;
m für eine ganze Zahl gleich null, 1, 2 oder 3 steht;
R¹³, R¹⁴, R¹⁵ und R¹⁶ jeweils unabhängig ausgewählt sind aus der Gruppe bestehend aus Wasserstoff; C₁₋₆-Alkyl; Aryl und Arylcarbonyl; oder R¹⁵ und R¹⁶ zusammen einen zweiwertigen Rest C₄₋₅-Alkandiyl bilden können;
R¹⁷ ausgewählt ist aus der Gruppe bestehend aus Wasserstoff; C₁₋₆-Alkyl; C₁₋₆-Alkylcarbonyl; Halogenmethylcarbonyl; C₁₋₆-Alkyloxycarbonyl; Aryl; Di(aryl)methyl; C₁₋₆-Alkyl substituiert durch Hydroxy, C₁₋₆-Alkyloxy, Carboxyl, C₁₋₆-Alkylcarbonyloxy, C₁₋₆-Alkyloxycarbonyl und Aryl;
R³ jeweils unabhängig ausgewählt ist aus der Gruppe bestehend aus Wasserstoff; Halogen; Cyano; Hydroxy; Halogenmethyl; Halogenmethoxy; Carboxyl; Nitro; Amino; Mono- oder Di(C₁₋₆-alkyl)amino; C₁₋₆-Alkylcarbonylamino; Aminosulfonyl; Mono- oder Di(C₁₋₆-alkyl)aminosulfonyl; C₁₋₆-Alkyl; C₁₋₆-Alkyloxy; C₁₋₆-Alkylcarbonyl und C₁₋₆-Alkyloxycarbonyl ;
R⁴ jeweils unabhängig ausgewählt ist aus der Gruppe bestehend aus Wasserstoff; Halogen; Cyano; Hydroxy; Halogenmethyl; Halogenmethoxy; Carboxyl; Nitro; Amino; Mono- oder Di(C₁₋₆-alkyl)amino; C₁₋₆-Alkylcarbonylamino; Aminosulfonyl; Mono- oder Di(C₁₋₆-alkyl)aminosulfonyl; C₁₋₆-Alkyl; C₁₋₆-Alkyloxy; C₁₋₆-Alkylcarbonyl und C₁₋₆-Alkyloxycarbonyl;
R⁵ jeweils unabhängig ausgewählt ist aus der Gruppe bestehend aus C₁₋₆-Alkyl; Cyano und Halogenmethyl;
R⁶ und R⁷ jeweils unabhängig voneinander ausgewählt sind aus der Gruppe bestehend aus Wasserstoff, Hydroxy, C₁₋₆-Alkyl, Halogenmethyl und C₁₋₆-Alkyloxy; mit der Maßgabe, daß R⁶ und R⁷ nicht gleichzeitig für Wasserstoff stehen; oder R⁶ und R⁷ zusammen einen Methylenrest (=CH₂) bilden können; oder zusammen mit dem Kohlenstoffatom, an das sie gebunden sind, ein Carbonyl bilden können; und
Aryl für Phenyl; oder Phenyl substituiert durch 1, 2 oder 3 Substituenten ausgewählt aus der Gruppe bestehend aus Halogen, Hydroxy, C₁₋₆-Alkyl und Halogenmethyl steht.

2. Verbindungen nach Anspruch 1, **dadurch gekennzeichnet, daß** n für 1 steht.

3. Verbindungen nach Anspruch 1 oder 2, **dadurch gekennzeichnet, daß**
n für eine ganze Zahl gleich 1 steht;
p für eine ganze Zahl gleich null oder 1 steht;
q für eine ganze Zahl gleich null oder 1 steht;
r für eine ganze Zahl gleich null steht;
R¹ und R² jeweils unabhängig ausgewählt sind aus der Gruppe bestehend aus Wasserstoff; C₁₋₆-Alkyl; Aryl; Alkylsulfonyl und C₁₋₆-Alkyl substituiert durch Carboxyl oder Aryl;
R³ ausgewählt ist aus der Gruppe bestehend aus Wasserstoff; Halogen; Amino; Mono- oder Di(C₁₋₆-alkyl)amino und C₁₋₆-Alkyloxy;
R⁴ für Wasserstoff oder Halogen steht;
R⁶ und R⁷ jeweils unabhängig voneinander ausgewählt sind aus der Gruppe bestehend aus Wasserstoff; Hydroxy; C₁₋₆-Alkyl und C₁₋₆-Alkyloxy; mit der Maßgabe, daß R⁶ und R⁷ nicht gleichzeitig für Wasserstoff stehen; oder R⁶ und R⁷ zusammen Methylen (=CH₂) bilden; oder zusammen mit dem Kohlenstoffatom, an das sie gebunden sind, ein Carbonyl bilden.

4. Verbindungen nach einem der Ansprüche 1 bis 3, **dadurch gekennzeichnet, daß** R¹ und R² jeweils unabhängig ausgewählt sind aus der Gruppe bestehend aus Wasserstoff; Methyl; Ethyl; Methoxy; Phenyl und Benzyl.

5. Verbindungen nach einem der Ansprüche 1 bis 4, **dadurch gekennzeichnet, daß** sowohl R¹ als auch R² für Methyl stehen; oder R¹ für Wasserstoff steht und R² für Methyl steht.

6. Verbindungen nach einem der Ansprüche 1 bis 5, **dadurch gekennzeichnet, daß** p für null oder 1 steht und R³ ausgewählt ist aus der Gruppe bestehend aus Wasserstoff; Fluor; Chlor; Brom; Methoxy; Amino; Methylamino und Dimethylamino.

7. Verbindungen nach einem der Ansprüche 1 bis 6, **dadurch gekennzeichnet, daß** q für null oder 1 steht und R⁴ ausgewählt ist aus der Gruppe bestehend aus Wasserstoff und Fluor.

8. Verbindungen nach einem der Ansprüche 1 bis 7, **dadurch gekennzeichnet, daß** R⁶ und R⁷ ausgewählt sind aus der Gruppe bestehend aus Wasserstoff, Methyl, Ethyl, Isopropyl, Hydroxy, Methoxy und Isopropoxy; oder R⁶ und R⁷ zusammen Methylen bilden können; oder zusammen mit dem Kohlenstoffatom, an das sie gebunden sind, ein Carbonyl bilden können.

9. Verbindungen nach einem der Ansprüche 1 bis 8, **dadurch gekennzeichnet, daß** die Wasserstoffatome an den Kohlenstoffatomen 3a und 12b eine trans-Konfiguration aufweisen, und Verbindungen nach einem der Ansprüche 1 bis 8, **dadurch gekennzeichnet, daß** die Verbindungen die (2α,3aα,12bβ)-stereochemische Konfiguration aufweisen.

10. Verbindungen nach einem der Ansprüche 1 bis 9, **dadurch gekennzeichnet, daß** die Verbindungen aus der folgenden Gruppe von Verbindungen ausgewählt sind:
ο (5,11-Difluor-8-methylen-3,3a,8,12b-tetrahydro-2H-1-oxa-dibenzo[e,h]azulen-2-ylmethyl)dimethylamin;
ο 11-Fluor-2-methylaminomethyl-3,3a,8,12b-tetrahydro-2*H*-1-oxadibenzo[e,h]azulen-8-ol;
ο 11-Fluor-8-methyl-2-methylaminomethyl-3,3a,8,12b-tetrahydro-2H-1-oxadibenzo[e,h]azulen-8-ol;
ο 2-Dimethylaminomethyl-8-methyl-3,3a,8,12b-tetrahydro-2*H*-1-oxadibenzo[e,h]azulen-8-ol;
ο (11-Fluor-8-methoxy-8-methyl-3,3a,8,12b-tetrahydro-2*H*-1-oxadibenzo[e,h]azulen-2-ylmethyl)dimethylamin;
ο (11-Fluor-8-methyl-3,3a,8,12b-tetrahydro-2*H-*1-oxadibenzo[e,h]azulen-2-ylmethyl)dimethylamin;
ο (8-Methyl-3,3a,8,12b-tetrahydro-2*H*-1-oxadibenzo[e,h]azulen-2-ylmethyl)dimethyl)-amin; und
ο (8-Methyl-3,3a,8,12b-tetrahydro-2*H*-1-oxadibenzo[e,h]azulen-2-ylmethyl)methylamin.

11. Verbindungen nach einem der Ansprüche 1 bis 10 zur Verwendung als Medizin.

12. Verwendung einer Verbindung nach einem der Ansprüche 1 bis 10 zur Herstellung eines Medikaments zur Behandlung von durch Serotonin, Dopamin und Norepinephrin vermittelten Leiden.

13. Verwendung einer Verbindung nach Anspruch 12 zur Herstellung eines Medikaments zur Behandlung und/oder Prophylaxe von Angst, Psychose, Depression, Migräne und süchtigmachenden Eigenschaften von Drogen.

14. Pharmazeutische Zusammensetzung, enthaltend einen pharmazeutisch unbedenklichen Träger und, als Wirkstoff, eine therapeutisch wirksame Menge einer Verbindung nach einem der Ansprüche 1 bis 10.

15. Verfahren zur Herstellung einer Verbindung nach Anspruch 14, **dadurch gekennzeichnet, daß** man einen pharmazeutisch unbedenklichen Träger innig mit einer therapeutisch wirksamen Menge einer Verbindung nach einem der Ansprüche 1 bis 10 mischt.

## Revendications

1. Composé de formule (I) 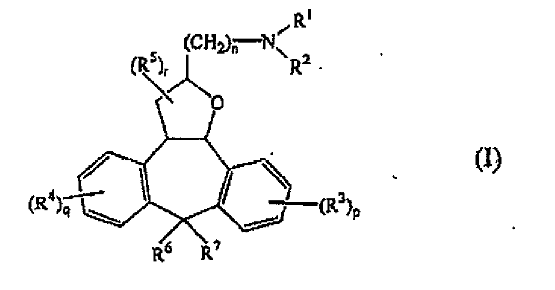 un sel d'addition à un acide ou une base pharmaceutiquement acceptable de celui-ci, une forme stéréochimiquement isomère de celui-ci et une forme N-oxyde de celui-ci, dans laquelle :
n est un entier valant zéro, 1, 2, 3, 4, 5 ou 6 ;
p est un entier valant zéro, 1, 2, 3 ou 4 ;
q est un entier valant zéro, 1 2, 3 ou 4 ;
r est un entier valant zéro, 1, 2, 3, 4 ou 5 ;
R¹ et R² sont, chacun indépendamment, choisis parmi le groupe constitué d'un atome d'hydrogène ; d'un groupe alkyle en C₁₋₆ ; d'un groupe C₁₋₆alkylcarbonyle ; d'un groupe halogénométhylcarbonyle ; d'un groupe aryle ; d'un groupe alkylsulfonyle et d'un groupe alkyle en C₁₋₆ substitué par un groupe hydroxy, un groupe alkyloxy en C₁₋₆, un groupe carboxyle, un groupe C₁₋₆alkylcarbonyloxy, un groupe C₁₋₆alkyloxycarbonyle ou un groupe aryle ;
ou R¹ et R², pris conjointement avec l'atome d'azote auquel ils sont fixés, peuvent former un noyau morpholinyle ou un radical de formules(a) à (e) : 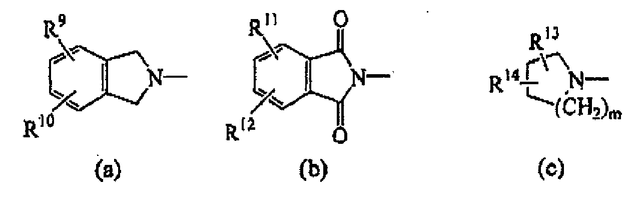 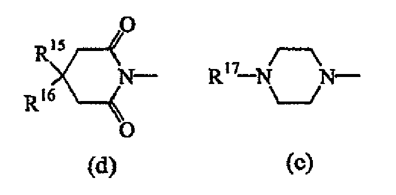 dans lesquelles :
R⁹, R¹⁰, R¹¹ et R¹² sont, chacun indépendamment, choisis parmi le groupe constitué d'un atome d'hydrogène ; d'un groupe halogéno ; d'un groupe halogénométhyle et d'un groupe alkyle en C₁₋₆ ;
m est un entier valant zéro, 1, 2 ou 3 ;
R¹³, R¹⁴, R¹⁵ et R¹⁶ sont, chacun indépendamment, choisis parmi le groupe constitué d'un atome d'hydrogène ; d'un groupe alkyle en C₁₋₆ ; d'un groupe aryle et d'un groupe arylcarbonyle ; ou R¹⁵ et R¹⁶, pris conjointement, peuvent former un radical bivalent alcanediyle en C₄₋₅ ;
R¹⁷ est choisi parmi le groupe constitué d'un atome d'hydrogène ; d'un groupe alkyle en C₁₋₆ ; d'un groupe C₁₋₆alkylcarbonyle ; d'un groupe halogénométhylcarbonyle ; d'un groupe C₁₋₆alkyloxycarbonyle ; d'un groupe aryle ; d'un groupe di(aryl)méthyle ; d'un groupe alkyle en C₁₋₆ substitué par un groupe hydroxy ; d'un groupe alkyloxy en C₁₋₆, d'un groupe carboxyle, d'un groupe C₁₋₆alkylcarbonyloxy, d'un groupe C₁₋₆alkyloxycarbonyle et d'un groupe aryle ;
chaque R³ est choisi indépendamment parmi le groupe constitué d'un atome d'hydrogène ; d'un groupe halogéno, d'un groupe cyano, d'un groupe hydroxy, d'un groupe halogénométhyle ; d'un groupe halogénométhoxy ; d'un groupe carboxyle ; d'un groupe nitro ; d'un groupe amino ; d'un groupe mono- ou di(C₁₋₆alkyl)amino; d'un groupe C₁₋₆alkylcarbonylamino ; d'un groupe aminosulfonyle ; d'un groupe mono- ou di(C₁₋₆alkyl)aminosulfonyle ; d'un groupe alkyle en C₁₋₆ ; d'un groupe alkyloxy en C₁₋₆ ; d'un groupe C₁₋₆alkylcarbonyle et d'un groupe C₁₋₆alkyloxycarbonyle ;
chaque R⁴ est choisi indépendamment parmi le groupe constitué d'un atome d'hydrogène ; d'un groupe halogéno, d'un groupe cyano, d'un groupe hydroxy, d'un groupe halogénométhyle ; d'un groupe halogénométhoxy ; d'un groupe carboxyle ; d'un groupe nitro ; d'un groupe amino ; d'un groupe mono- ou di(C₁₋₆alkyl)amino ; d'un groupe C₁₋₆alkylcarbonylamino ; d'un groupe aminosulfonyle ; d'un groupe mono- ou di(C₁₋₆alkyl)aminosulfonyle ; d'un groupe alkyle en C₁₋₆ ; d'un groupe alkyloxy en C₁₋₆ ; d'un groupe C₁₋₆alkylcarbonyle et d'un groupe C₁₋₆alkyloxycarbonyle ;
chaque R⁵ est choisi indépendamment parmi le groupe constitué d'un groupe alkyle en C₁₋₆ ; d'un groupe cyano et d'un groupe halogénométhyle ;
R⁶ et R⁷ sont choisis, chacun indépendamment l'un de l'autre, parmi le groupe constitué d'un atome d'hydrogène, d'un groupe hydroxy, d'un groupe alkyle en C₁₋₆, d'un groupe halogénométhyle et d'un groupe alkyloxy en C₁₋₆ ; à condition que R⁶ et R⁷ ne soient pas simultanément un atome d'hydrogène ; ou R⁶ et R⁷, pris conjointement, peuvent former un radical méthylène (=CH₂) ; ou, conjointement avec l'atome de carbone auquel ils sont fixés, un groupe carbonyle ; et
aryle est un groupe phényle ; ou un groupe phényle substitué par 1, 2 ou 3 substituants choisis parmi le groupe constitué d'un groupe halogéno, d'un groupe hydroxy, d'un groupe alkyle en C₁₋₆ et d'un groupe halogénométhyle.

2. Composé selon la revendication 1, **caractérisé en ce que** n vaut 1.

3. Composé selon l'une quelconque des revendications 1 à 2, **caractérisé en ce que**
n est un entier valant 1 ;
p est un entier valant zéro ou 1 ;
q est un entier valant zéro ou 1 ;
r est un entier valant zéro ;
R¹ et R² sont choisis, chacun indépendamment, parmi le groupe constitué d'un atome d'hydrogène, d'un groupe alkyle en C₁₋₆ ; d'un groupe aryle ; d'un groupe alkylsulfonyle et d'un groupe alkyle en C₁₋₆ substitué par un groupe carboxyle ou un groupe aryle ;
R³ est choisi parmi le groupe constitué d'un atome d'hydrogène ; d'un groupe halogéno ; d'un groupe amino ; d'un groupe mono- ou d'un groupe di(C₁₋₆alkyl)amino et d'un groupe alkyloxy en C₁₋₆;
R⁴ est un atome d'hydrogène ou un groupe halogéno ;
R⁶ et R⁷ sont choisis, chacun indépendamment l'un de l'autre, parmi le groupe constitué d'un atome d'hydrogène, d'un groupe hydroxy, d'un groupe alkyle en C₁₋₆ et d'un groupe alkyloxy en C₁₋₆ ; 1 à condition que R⁶ et R⁷ ne soient pas simultanément un atome d'hydrogène ; ou R⁶ et R⁷, pris conjointement, forment un radical méthylène (=CH₂) ; ou, conjointement avec l'atome de carbone auquel ils sont fixés, un groupe carbonyle.

4. Composé selon l'une quelconque des revendications 1 à 3, **caractérisé en ce que** R¹ et R² sont choisis, chacun indépendamment, parmi le groupe constitué d'un atome d'hydrogène ; d'un groupe méthyle ; d'un groupe éthyle ; d'un groupe méthoxy ; d'un groupe phényle et d'un groupe benzyle.

5. Composé selon l'une quelconque des revendications 1 à 4, **caractérisé en ce que** R¹ et R² sont tous les deux un groupe méthyle ; ou R¹ est un atome d'hydrogène et R² est un groupe méthyle.

6. Composé selon l'une quelconque des revendications 1 à 5, **caractérisé en ce que** p vaut zéro ou 1 et R³ est choisi parmi le groupe constitué d'un atome d'hydrogène ; d'un groupe fluoro ; d'un groupe chloro ; d'un groupe bromo ; d'un groupe méthoxy ; d'un groupe amino ; d'un groupe méthylamino et d'un groupe diméthylamino.

7. Composé selon l'une quelconque des revendications 1 à 6, **caractérisé en ce que** q vaut zéro ou 1 et R⁴ est choisi parmi le groupe constitué d'un atome d'hydrogène et d'un groupe fluoro.

8. Composé selon l'une quelconque des revendications 1 à 7, **caractérisé en ce que** R⁶ et R⁷ sont choisis parmi le groupe constitué d'un atome d'hydrogène, d'un groupe méthyle, d'un groupe éthyle, d'un groupe isopropyle, d'un groupe hydroxy, d'un groupe méthoxy et d'un groupe isopropoxy ; ou R⁶ et R⁷, pris conjointement, peuvent former un groupe méthylène ; ou, conjointement avec l'atome de carbone auquel ils sont fixés, un groupe carbonyle.

9. Composé selon l'une quelconque des revendications 1 à 8, **caractérisé en ce que** les atomes d'hydrogène sur les atomes de carbone 3a et 12b présentent une configuration trans, ou composé selon l'une quelconque des revendications 1 à 8, **caractérisé en ce que** le composé présente la configuration stéréochimique (2α, 3aα, 12bβ).

10. Composé selon l'une quelconque des revendications 1 à 9, **caractérisé en ce que** les composés sont choisis parmi le groupe de composés :
ο (5,11-difluroro-8-méthylène-3,3a,8,12b-tétrahydro-2*H*-1-oxa-dibenzo-[e,h]azulén-2-ylméthyl)-diméthylamine ;
ο 11-fluoro-2-méthylaminométhyl-3,3a,8,12b-tétrahydro-2H-1-oxa-dibenzo[e,h]azulén-8-o1 ;
ο 11-fluoro-8-méthyl-2-méthylaminométhyl-3,3a,8,12b-tétrahydro-2H-1-oxa-dibenzo[e,h]azulén-8-o1 ;
ο 2-diméthylaminométhyl-8-méthyl-3,3a,8,12b-tétrahydro-2*H*-1-oxa-dibenzo[e,h]azulén-8-ol ;
ο (11-fluoro-8-méthoxy-8-méthyl-3,3a,8,12b-tétrahydro-2*H*-1-oxa-dibenzo[e,h]azulén-2-ylméthyl)-diméthyl-amine ;
ο (11-fluoro-8-méthyl-3,3a,8,12b-tétrahydro-2*H*-1-oxa-dibenzo[e,h]azulén-2-ylméthyl)-diméthyl-amine ;
ο (8-méthyl-3,3a,8,12b-tétrahydro-2H-1-oxa-dibenzo[e,h]azulén-2-ylméthyl)-diméthyl-amine ; et
ο (8-méthyl-3,3a,8,12b-tétrahydro-2H-1-oxa-dibenzo[e,h]azulén-2-ylméthyl)-méthyl-amine.

11. Composé selon l'une quelconque des revendications 1 à 10, destiné à être utilisé en tant que médicament.

12. Utilisation d'un composé selon l'une quelconque des revendications 1 à 10, pour la fabrication d'un médicament destiné au traitement des pathologies à médiation par la sérotonine, la dopamine et la norépinéphrine.

13. Utilisation d'un composé selon la revendication 12, pour la fabrication d'un médicament destiné au traitement et/ou à la prophylaxie de l'anxiété, de la psychose, de la dépression, de la migraine et de propriétés addictives de drogues d'abus.

14. Composition pharmaceutique comprenant un support pharmaceutiquement acceptable et, en tant qu'ingrédient actif, une quantité thérapeutiquement efficace d'un composé selon l'une quelconque des revendications 1 à 10.

15. Procédé de préparation d'une composition selon la revendication 14, **caractérisé en ce qu'**un support pharmaceutiquement acceptable est mélangé intimement avec une quantité thérapeutiquement efficace d'un composé selon l'une quelconque des revendications 1 à 10.
